# EUROPEAN PATENT SPECIFICATION

(11) **EP 2 373 650 B1**
(45) Date of publication and mention of the grant of the patent: **24.10.2012**
(21) Application number: 09765124.4
(22) Date of filing: 10.12.2009
(51) Int. Cl.: C07D 471/04, A61K 31/4745

(54) **TRIPLE SUBSTITUTED PHENANTHROLINE DERIVATIVES FOR THE TREATMENT OF NEURODEGENERATIVE OR HAEMATOLOGICAL DISEASES OR CONDITIONS, OR CANCER**
DREIFACH SUBSTITUIERTE PHENANTHROLINDERIVATE ZUR BEHANDLUNG NEURODEGENERATIVER ODER HÄMATOLOGISCHER KRANKHEITEN BZW. LEIDEN, ODER VON KREBS
DÉRIVÉS DE LA PHÉNANTHROLINE À TRIPLE SUBSTITUTION POUR LE TRAITEMENT DE MALADIES OU CONDITIONS NEURODÉGÉNÉRATIVES OU HÉMATOLOGIQUES, OU DU CANCER

(30) Priority: 10.12.2008 EP 08382073
(43) Date of publication of application: 12.10.2011
(73) Proprietor: Noscira, S.A., 28760 Tres Cantos (ES)
(72) Inventor: MEDINA PADILLA, Miguel, E-28760 Tres Cantos (Madrid) (ES); CASTRO MORERA, Ana, E-28760 Tres Cantos (Madrid) (ES); SÁNCHEZ-QUESADA, Jorge, E-28760 Tres Cantos (Madrid) (ES); GARCÍA PALOMERO, Esther, E-28760 Tres Cantos (Madrid) (ES); ALONSO CASCÓN, Mercedes., E-28760 Tres Cantos (Madrid) (ES); HERRERO SANTOS, Susana., E-28760 Tres Cantos (Madrid) (ES); VELA RUIZ, Marta., E-28760 Tres Cantos (Madrid) (ES); USÁN EGEA, Paola., E-28760 Tres Cantos (Madrid) (ES); RODRIGUEZ VILLANUEVA, Ana Luisa., E-28760 Tres Cantos (Madrid) (ES)
(74) Representative: ABG Patentes, S.L.
(86) International application number: PCT/EP2009/066817
(87) International publication number: WO 2010/066832

(56) References cited:
- CARAGOUNIS, APHRODITE ET AL: "Differential modulation of Alzheimer's disease amyloid .beta.-peptide accumulation by diverse classes of metal ligands" BIOCHEMICAL JOURNAL, CODEN: BIJOAK; ISSN: 0264-6021, vol. 407, no. 3, 1 November 2007 (2007-11-01), pages 435-450, XP002528830
- GAETA A ET AL: "The crucial role of metal ions in neurodegeneration: the basis for a promising therapeutic strategy" BRITISH JOURNAL OF PHARMACOLOGY, NATURE PUBLISHING GROUP, BASINGSTOKE, HANTS, vol. 146, no. 8, 1 December 2005 (2005-12-01), pages 1041-1059, XP002456896 ISSN: 0007-1188
- MLOCHOWSKI J ET AL: "THE SYNTHESIS OF PHENANTHROLINE ANALOGUES OF CAERULOMYCIN" ROCZNIKI CHEMII ANNALES SOCIETATIS CHIMICAE POLONORUM,, vol. 45, 1 January 1971 (1971-01-01), pages 803-807, XP008084415 ISSN: 0035-7677

## Description

### FIELD OF THE INVENTION

The present invention relates to the use of some triple substituted phenanthroline derivatives for the treatment and/or prophylaxis of a neurodegenerative or haematological disease or condition, particularly Alzheimer's disease (AD), or cancer. Additionally, new triple substituted phenanthroline derivatives are provided, processes for preparing such derivatives and pharmaceutical compositions comprising them.

### BACKGROUND OF THE INVENTION

AD and Parkinson's disease (PD) are the most frequent progressive neurodegenerative diseases affecting millions of people in the world. Because a significant percentage of patients share common clinical and pathological symptoms from both entities, this seems to indicate the existence of a common pathological mechanism.

Oxidative stress is known to be involved in many diseases, including atherosclerosis, Parkinson's disease and AD, and may be also important in ageing and cancer.

Reactive oxygen species (ROS), such as oxygen radical superoxide (O₂⁻) or hydrogen peroxide (H₂O₂), are produced during normal metabolic processes and perform several useful functions (Reactive oxygen species and the central nervous system, Halliwell B., J. Neurochem., 1992, 59, 1609-1623)*.* Cells are provided with several mechanisms to control levels of these oxidative agents, for instance, superoxide dismutase (SOD), glutathione or vitamin E. In normal physiological conditions, a balance between ROS and these anti-oxidative mechanisms exists. An excessive production of ROS and a loss of efficiency of the anti-oxidative defences can lead to cellular oxidative stress and thus to pathological conditions in cells and provoke tissue damage. This event seems to occur more dramatically in neurons, because of their high rate of metabolic activity, and thus seems to be related to a series of degenerative processes, diseases and syndromes, for example, AD, PD, amyotrophic lateral sclerosis (ALS) and schizophrenia (Glutathione, oxidative stress and neurodegeneration, Schulz et al., Eur. J. Biochem., 2000, 267, 4904-4911)*.* Also other diseases or pathological conditions have been related to oxidative stress, such as Huntington's Disease (Oxidative damage in Huntington's disease, Segovia J. and Pérez-Severiano F, Methods Mol. Biol., 2004, 207, 321-334), brain injuries, such as stroke and ischemia, (Oxidative Stress in the Context of Acute Cerebrovascular Stroke, El Kossi et al., Stroke, 2000, 31, 1889-1892), diabetes (Oxidative stress as a therapeutic target in diabetes: revisiting the controversy, Wiernsperger NF, Diabetes Metab., 2003, 29, 579-85), multiple sclerosis (The role of oxidative stress in the pathogenesis of multiple sclerosis: the need for *effective antioxidant therapy, Gilgun-Sherki Y. et al., J. Neurol., 2004, 251 (3), 261-8), epilepsy (Oxidative injury in epilepsy: potential for antioxidant therapy?, Costello D.J. and Delanty N., Expert. Rev. Neurother., 2004, 4(3), 541-553), atherosclerosis (The oxidative stress hypothesis of atherogenesis, luliano L., Lipids, 2001, 36 suppl, S41-44)*,* Friedreich's Ataxia (Oxidative stress, mitochondrial dysfuntion and cellular stress response in Friedreich's ataxia, Calabrese et al., J. Neurol. Sci., 2005, 233, 145-162),heart failure (Oxygen, oxidative stress, hypoxia and heart failure, Giordano F.J. et al., J. Clin. Invest,; 2005, 115(3), 500-508), cancer (ROS stress in cancer cells and therapeutic implications, Pelicano et al., Drug Resistance Updates, 2004, 7(2), 97-110) and tumor progression (The signaling mechanism of ROS in tumor progression, Wu W.S., Cancer and metastasis reviews, 2006, 25(4), 695-705). Treatments that lead to an enhancement of the anti-oxidative mechanisms may slow down the progression of some of the mentioned diseases.

Another type of cellular stress is the endoplasmic reticulum (ER) stress. The ER is an intracellular organelle represented by an extensive network formed by cistemae and microtubules and which extends from the nuclear envelope to the cell surface in all eukaryotic cells. ER plays several vital functions: the rough ER is the place for protein synthesis and postranslational changes for the correct folding of proteins, ER is the common transport route to deliver proteins to their proper destination within the cell and it is also a Ca²⁺ reservoir. Disturbances in the function of ER lead to accumulation of unfolded proteins within the ER, inducing a condition generally referred to as ER stress. These disturbances can be caused not only by biochemical imbalance but also by disturbance in the ER Ca²⁺ homeostasis. Some studies (Glycogen synthase kinase 3β (GSK3β) mediates 6-hydroxydopamine-induced neuronal death, Chen et al., FASEB J. 2004, 18(10), 1162-4) demonstrate that ER stress activates the enzyme glycogen synthase kinase 3β, an enzyme involved in the neurodegenerative process occurred in patients with AD.

Tumor cell demise is an important event in the elimination of abnormal malignant cells and provides an important mechanism of natural tumor suppression. Abnormalities incapacitating these finely tuned processes provide a strong advantage for cancer clones to succeed in evading both the physiological control systems and therapeutic intervention. Although currently available data indicate that elimination of malignant cells often depends on classical apoptotic pathways (mitochondrial and/or death-receptor pathways), the evidence is mounting that alternative apoptotic and non-apoptotic pathways may effectively contribute to tumor cell death. Among these mechanisms, experimental evidence indicates that ER and Golgi apparatus can activate both pro-survival (recovery) mechanisms as well as cell suicide programs if the stress-signalling threshold is exceeded. It is thus conceivable that the fragile balance of protein trafficking between various subcellular compartments provides an exceptional therapeutic opportunity (ER-Golgi network--a future target for anti-cancer therapy, Wlodkowic D. et al., 2009, Leukemia Research, 33 (11), 1440-1447)*.*

The catecholaminergic neurotoxin 6-hydroxydopamine (6-OHDA) is formed endogenously in patients suffering from Parkinson's disease. 6-OHDA has two ways of action: it easily forms free radicals and it is a potent inhibitor of the mitochondrial respiratory chain complexes I and IV. 6-OHDA models are used to produce a broad spectrum of neurochemical and behavioural deficits characterising DA degeneration in humans, specially for PD (e.g. Mechanism of 6-hydroxydopamine neurotoxicity, Glinka Y et al., J Neural Transm Suppl., 1997, 50, 55-66; The implementation of acute versus chronic animal models for treatment discovery in Parkinson's disease, Wills GL et al., Rev Neurosci., 2004, 15(1), 75-87).

A common sign of neurodegenerative diseases is the accumulation and deposits of misfolded proteins which affect several signalling pathways which lead finally to neuronal death. Some authors (ER stress and neurodegenerative diseases, Lindholm et al., Cell Death and Differentiation, 2006, 13, 385-392) consider that ER stress is related to several neurodegenerative diseases such as, PD, AD, ALS, and transmissible spongiform encephalopaties (TSEs).

In view of the above, an interesting approach for developing new pharmaceutical compounds for treating neurodegenerative diseases may be designing compounds which inhibit cellular oxidative stress.

Amyloid beta (Aβ) is a peptide that is the main constituent of amyloid plaques in the brains of AD patients. Similar plaques appear in some variants of Lewy body dementia and in inclusion body myositis, a muscle disease. Aβ also forms aggregates coating cerebral blood vessels in cerebral amyloid angiopathy.

Aβ is formed after sequential cleavage of the amyloid precursor protein (APP) by the β- and γ-secretases. Either Aβ₄₂ or Aβ₄₀ are produced depending on where the cleavage occurs. APP is a transmembrane glycoprotein. Autosomal-dominant mutations in APP cause hereditary early-onset AD, likely as a result of altered proteolytic processing. Increases in total Aβ levels have been implicated in the pathogenesis of both familial and sporadic AD (Soluble Amyloid ß Peptide Concentration as a Predictor of Synaptic Change in Alzheimer's Disease, L. et al., The American Journal of Pathology,1999, Lue, L 155(3), 853-662 ).

According to the "amyloid hypothesis", accepted by the majority of researchers, the plaques are responsible for the pathology of AD. Intra-cellular deposits of tau protein are also seen in the disease, and may also be implicated. The oligomers that form on the amyloid pathway, rather than the mature fibrils, may be the cytotoxic species.

Thus, the development of inhibitors of amyloid beta secretion are a current strategy to find treatments for diseases in which amyloidosis is involved, such as AD, PD, Huntington's disease, TSEs, Prion diseases, Creutzfeldt-Jakob disease and Bovine spongiform encephalopathy.

On the other hand, iron chelators are used to treat some kinds of haematological diseases, such as thalassaemia, anaemia, aplastic anaemia, myelodysplastic syndrome, diabetes, Diamond-Blackfan anaemia, sickle cell disease, hematologic disorders which require regular red cell transfusions, iron-induced cardiac dysfunction, and iron-induced heart failure.

Metals such as iron are capable of redox cycling in which a single electron may be accepted or donated by the metal. This action catalyzes reactions that produce reactive radicals and can produce reactive oxygen species. The most important reactions are probably Fenton's reaction and the Haber-Weiss reaction, in which hydroxyl radical is produced from reduced iron and hydrogen peroxide. The hydroxyl radical then can lead to modifications of amino acids (e.g. meta-tyrosine and ortho-tyrosine formation from phenylalanine), carbohydrates, initiate lipid peroxidation, and oxidize nucleobases. Most enzymes that produce reactive oxygen species contain one of these metals. The presence of such metals in biological systems in an uncomplexed form (not in a protein or other protective metal complex) can significantly increase the level of oxidative stress. Therefore, it is desirable that chelating ligands for the treatment of conditions according to the invention, show a preference towards Fe(II) rather than Fe(III).

Iron chelators deferoxamine and deferiprone, have been used in humans since the 1970s and the late 1980s, respectively, and lately a new drug, deferasirox has been used in humans. Deferoxamine has proven efficient in thalassemia major, sickle cell disease and other hematologic disorders for which hematologic disorders, but can only be administered subcutaneously (Oral chelators deferasirox and deferiprone for transfusional iron overload in thalassemia major: new data, new questions, Neufeld E.L., Blood, 2006, 107(9), 3436-3441). Deferasirox, approved in the US for chronic iron overload due to blood transfusions, has shown moderate to good success N(ew Advances in Iron Chelation Therapy, Cohen, A.R., Hematology, 2006, 42-47). Combination therapy with deferiprone and deferoxamine is also being used.

However, side effects have been associated with the use of these drugs; deferiprone often causes gastrointestinal symptoms, erosive arthritis, neutropenia and in some cases agranulocytosis; deferiprone therapy requires weekly complete blood count and ancillary supplies for infusion, so close monitoring is required; deferoxamine presents gastrointestinal symptoms and joint pain and deferasirox is costly. Therefore there still remains a need for additional therapeutic iron chelators for use in these hematological diseases, produced and used with low cost and reduced side effects.

Additionally, chelating drugs and chelator metal complexes have been described as useful agents for the prevention, diagnosis and treatment of cancer. Cancer cells and normal cells require essential metal ions such as iron, copper and zinc for growth and proliferation. Chelators can target the metabolic pathways of cancer cells through the control of proteins involved in the regulation of these metals and also of other molecules involved in cell cycle control, angiogenesis and metastatic suppression. Other targets include the inhibition of specific proteins such as ribonucleotide reductase involved in DNA synthesis, the inhibition of free radical damage on DNA caused by iron and copper catalytic centers, the inhibition of microbial growth in immuno compromised cancer patients and the decorporation of radioactive and other toxic metals causing cancer. Although many experimental chelators have been shown to be effective as anti-cancer agents, only a few, e.g., dexrazoxane, deferoxamine (DFO) and triapine, have reached the stage of clinical testing or application (Chelators controlling metal metabolism and toxicity pathways: applications in cancer prevention, diagnosis and treatment, Kontoghiorghes G.J. et al., Hemoglobin, 2008, 32(1-2), 217-27).

It is well known that phenanthroline derivatives exhibit good iron chelating properties. Some phenanthroline derivatives are shown in patent PL76345. It would be highly recommended to find new phenanthroline derivatives which can show improved properties in chelating iron metal in order to provide an enhanced capability for treating the haematological mentioned diseases and for preventing, diagnosing and treating cancer.

### SUMMARY OF THE INVENTION

The authors of the present invention have found a new family of compounds, namely triple substituted phenanthroline derivatives, defined by formula (I) as detailed below, which show the properties of protecting from oxidative stress, particularly from hydrogen peroxide-cell death and 6-hydroxydopamine-cell death, having a neuroprotective effect against A toxicity, and inhibiting A secretion. They may thus be useful for the treatment or prophylaxis of neurodegenerative diseases or conditions. In addition, these compounds are characterized for acting as specific iron (II) chelators and therefore they could also be used to treat haematological diseases and cancer. Further, the compounds do not affect cell viability at concentrations well above the active ones.

Therefore, according to a first aspect, the present invention is directed to a 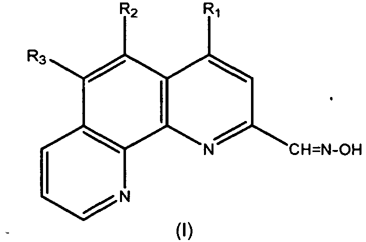 compound of formula (I):
wherein
R¹ is selected from -O-R⁴ and -S-R⁵, wherein R⁴ and R⁵ are selected from H and C₁-C₆ alkyl,
R² is selected from hydrogen, halogen, C₁-C₆-alkoxyl, C₁-C₆ alkyl and -O-(CH₂)ₙ-O-R⁶, wherein n is selected from 1, 2, 3, 4, 5, 6, and R⁶ is C₁-C₆ alkyl,
R³ is selected from hydrogen and C₁-C₆ alkoxyl, with the proviso that one of R² and R³ is H and the other is different from H, or any salt or solvate or stereoisomer or tautomer thereof.

According to a further aspect, the present invention is directed to the use of a compound of formula (I): 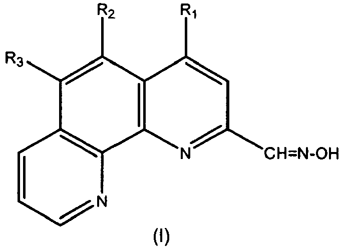 wherein
R¹ is selected from -O-R⁴ and -S-R⁵, wherein R⁴ and R⁵ are selected from H and C₁-C₆alkyl,
R² is selected from hydrogen, halogen, C₁-C₆-alkoxyl, C₁-C₆alkyl and -O-(CH₂)ₙ-O-R⁶, wherein n is selected from 1, 2, 3, 4, 5, 6, and R⁶ is C₁-C₆alkyl,
R³ is selected from hydrogen and C₁-C₆ alkoxyl,
with the proviso that one of R² and R³ is H and the other is different from H,
or any salt or solvate or stereoisomer or tautomer thereof,
in the preparation of a medicament for the treatment or prophylaxis of a neurodegenerative or haematological disease or condition or cancer.

A further aspect of the invention are the compounds of formula (I) as defined above for use in the treatment or prophylaxis of a neurodegenerative or haematological disease or condition or cancer.

The compounds of formula (I) may be used in biological assays wherein beta-amyloid secretion needs to be modulated. Therefore, in another aspect, the invention refers to the use of a compound of formula (I) as defined above, or any salt or solvate thereof, as reagent for biological assays, preferably as a reactive for pharmacokinetic assays, blood brain barrier crossing assays, chelation assays, for essays on protection against hydrogen peroxide-induced cell death, protection against 6-OHDA-induced cell death, neuroprotection against Aβ toxicity and inhibition of beta-amyloid secretion.

A further aspect of the invention refers to a method of treating or preventing a neurodegenerative or haematological disease or condition or cancer, said method comprising administering to a patient in need of such a treatment a therapeutically effective amount of at least one compound of formula (I) as defined above, its salts, solvates, stereoisomers or tautomers thereof, or a pharmaceutical composition thereof.

Another aspect of the present invention refers to a pharmaceutical composition comprising at least one compound of formula (I) as defined above, its salts or solvates or stereoisomers or tautomers thereof, and at least one pharmaceutically acceptable carrier.

According to a further aspect, the present invention is directed to a compound of formula (I) as defined above, its salts, solvates or stereoisomers or tautomers thereof, for use as a medicament.

According to a further aspect, the present invention is directed to a process for the synthesis of the compounds of formula I, its salts or solvates or stereoisomers or tautomers thereof.

### DETAILED DESCRIPTION OF THE INVENTION

In the above definition of compounds of formula (I) the following terms have the meaning indicated:
"C₁-C₆ Alkyl" refers to a linear or branched hydrocarbon chain radical consisting of carbon and hydrogen atoms, containing no unsaturation, having one to six carbon atoms, preferably one to three, and which is attached to the rest of the molecule by a single bond, e. g., methyl, ethyl, n-propyl, i-propyl, n-butyl, t-butyl, n-pentyl, etc.
"C₁-C₆ alkoxyl" refers to a radical of the formula -ORₐ where Rₐ is a "C₁-C₆ alkyl" radical as defined above, e. g., methoxy, ethoxy, propoxy, etc.
"Halogen" refers to bromo, chloro, iodo or fluoro.

### Compounds of formula I

An embodiment of the invention is directed to a compound of formula (I) wherein
R¹ is selected from -O-R⁴ and -S-R⁵, wherein R⁴ and R⁵ are selected from H and C₁-C₆ alkyl,
R² is selected from hydrogen, halogen, C₁-C₆-alkoxyl, C₁-C₆ alkyl and -O-(CH₂)ₙ-O-R⁶, wherein n is selected from 1, 2, 3, 4, 5, 6, and R⁶ is C₁-C₆alkyl,
R³ is selected from hydrogen and C₁-C₆ alkoxyl,
with the proviso that one of R² and R³ is H and the other is different from H,
or any salt or solvate or stereoisomer or tautomer thereof.

According to a preferred embodiment, R⁴ and R⁵ are selected from H and methyl.

According to another preferred embodiment, R² is selected from hydrogen, fluor, methyl, methoxy and -O-(CH₂)₂-O-CH₃.

According to a further preferred embodiment, R³ is selected from hydrogen and methoxyl.

Preferably, the double bond of the oxime group -CH=NOH presents E conformation, according to the following structural formula (Ia):

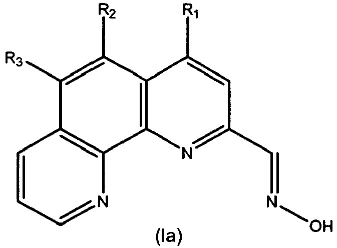

Preferably, the compound of formula (I) is selected from the following compounds:
5-Methoxy-4-methylsulfanyl-[1,10]phenanthroline-2-carbaldehyde oxime
5-Fluoro-4-methoxy-[1,10]phenanthroline-2-carbaldehyde oxime
4-Methoxy-5-(2-methoxy-ethoxyh[1,10]phenanthroline-2-carbaldehyde oxime
5-Methyl-4-methylsulfanyl-[1,10]phenanthroline-2-carbaldehyde oxime
6-Methoxy-4-methylsulfanyl-[1,10]phenanthroline-2-carbaldehyde oxime
4-Hydroxy-6-methoxy-[1,10]phenanthroline-2-carbaldehyde oxime

The compounds of formula (I) may be in the form of salts, preferably pharmaceutically acceptable salts, or in the form of solvates. The term "pharmaceutically acceptable salts" refers to any salt which upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. Preferably, "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate. Preferably, the solvates are pharmaceutically acceptable solvates.

The preparation of salts and solvates can be carried out by methods known in the art. For instance, pharmaceutically acceptable salts of compounds provided herein are synthesized from the parent compound, which contains a basic moiety, by conventional chemical methods. Generally, such salts are, for example, prepared by reacting the free base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate.

One preferred pharmaceutically acceptable form is the crystalline form, including such form in a pharmaceutical composition. In the case of salts and solvates the additional ionic and solvent moieties must also be non-toxic. The compounds of the invention may present different polymorphic forms, it is intended that the invention encompasses all such forms.

The compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or a nitrogen by ¹⁵N-enriched nitrogen are within the scope of this invention.

The compounds of the present invention represented by the above described formula (I) may include enantiomers or diastereomers depending on the presence of chiral centres or isomers depending on the presence of multiple bonds (e.g. Z, E). The single isomers, enantiomers or diastereoisomers and mixtures thereof fall within the scope of the present invention.

### Uses of compounds of formula (I)

Within the frame of the present invention, the expression "neurodegenerative disease or condition" means any disease or condition in which neurodegeneration occurs. Such disease or condition includes, but is not limited to, any disease or condition selected from Alzheimer's disease, Parkinson's disease, amyotrophic lateral sclerosis (ALS), schizophrenia, Huntington's Disease, brain injuries, such as stroke and ischemia, multiple sclerosis, epilepsy, Friedreich's Ataxia, spongiform encephalopaties, amyloidosis, vascular dementia, tauophaties, progressive supranuclear palsy, frontotemporal lobular degeneration, subacute sclerosing panencephalitic parkinsonism, postencephalitic parkinsonism, pugilistic encephalitis, guam parkinsonism-dementia complex, Pick's disease, corticobasal degeneration, frontotemporal dementia, AIDS associated dementia, multiple sclerosis, mood disorders such as depression, schizophrenia and bipolar disorders, promotion of functional recovery post stroke and brain injury, especially traumatic brain injury. In a preferred aspect of the invention, the neurodegenerative disease or condition is Alzheimer's Disease.

Within the frame of the present invention, the expression "haematological disease or condition" means any disease or condition in which disorders of the blood and blood forming tissues occurs. In a preferred embodiment, the haematological disease or condition is selected from thalassaemia, anaemia, aplastic anaemia, Diamond-Blackfan anemia, sickle cell disease, hematologic disorders which require regular red cell transfusions, myelodysplastic syndrome, iron-induced cardiac dysfunction, iron-induced heart failure, and diabetes, more preferably from thalassaemia, anaemia, aplastic anaemia, myelodysplastic syndrome and diabetes.

Within the frame of the present invention, the expression "cancer" includes intestines, liver, gastric, breast, lung, ovary, prostate, brain glioma, lymph, skin, pigment, thyroid gland, leukemia and multiple bone marrow cancer.

According to a preferred embodiment, R⁴ and R⁵ are selected from H and methyl.

According to another preferred embodiment, R² is selected from hydrogen, fluor, methyl, methoxy and -O-(CH₂)₂-O-CH₃.

According to a further preferred embodiment, R³ is selected from hydrogen and methoxyl.

Preferably, the double bond of the oxime group -CH=NOH presents E conformation, according to the following structural formula (Ia):

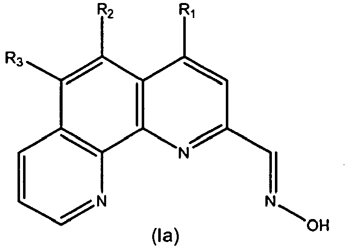

In a particular aspect, the compound of formula (I) used in the present invention is selected from the following compounds:
5-Methoxy-4-methylsulfanyl-[1,10]phenanthroline-2-carbaldehyde oxime
5-Fluoro-4-methoxy-[1,10]phenanthroline-2-carbaldehyde oxime
4-Methoxy-5-(2-methoxy-ethoxy)-[1,10]phenanthroline-2-carbaldehyde oxime
5-Methyl-4-methylsulfanyl-[1,10]phenanthroline-2-carbaldehyde oxime
6-Methoxy-4-methylsulfanyl-[1,10]phenanthroline-2-carbaldehyde oxime
4-Hydroxy-6-methoxy-[1,10]phenanthroline-2-carbaldehyde oxime

The compounds used in the present invention may be used with at least one other drug to provide a combination therapy. The at least other drug may form part of the same composition, or be provided as a separate composition for administration at the same time or at different time.

According to a further aspect, the present invention is directed to a method of treating or preventing a neurodegenerative or haematological disease or condition or cancer, said method comprises administering to a patient in need of such a treatment a therapeutically effective amount of at least one compound of formula (I), its salts or solvates, stereoisomers or tautomers thereof, as defined above or a pharmaceutical composition thereof.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

### Pharmaceutical compositions

According to a further aspect, the present invention is directed to a pharmaceutical composition comprising at least one compound of formula (I) as defined above, its salts or solvates or stereoisomers or tautomers thereof, and at least one pharmaceutically acceptable carrier.

As indicated earlier, the composition may further contain at least one other drug, to provide a combination therapy. The at least other drug may be any biologically active compound, especially any other drug known to be useful for treating neurodegenerative or haematological diseases or conditions, or cancer.

The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 1995.

Preferably, the carriers of the invention are approved by a regulatory agency of the Federal or a state government or listed in the U.S. Pharmacopeia or other generally recognized pharmacopeia for use in animals, and more particularly in humans

The carriers and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993.

Examples of pharmaceutical compositions include any solid (tablets, pills, capsules, granules etc.) or liquid (solutions, suspensions or emulsions) compositions for oral, topical or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

The compounds or compositions of the present invention may be administered by any suitable method, such as intravenous infusion, oral preparations, and intraperitoneal and intravenous administration. Oral administration is preferred because of the convenience for the patient and the chronic character of many of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.01 to 1000 mg/kg/day.

According to a further aspect, the present invention is directed to a compound of formula (I), its salts or solvates or stereoisomers or tautomers thereof, as defined above, for use as a medicament.

### Synthesis of compounds of formula (I)

The compounds of the present invention can be prepared following the general scheme shown below:

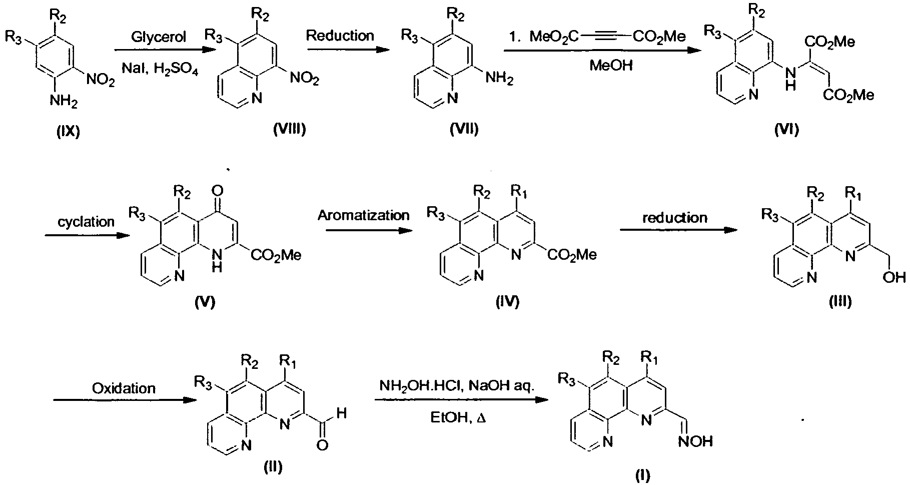

Thus, the compounds of the present invention may be prepared by a combination of reactions known in the art.

In a particular embodiment, the compounds of formula (I) can be prepared by a process comprising converting the aldehyde group -CHO in the compound of formula (II) into an oxime group, in the presence of hydroxylamine. The reaction preferably takes place in a polar protic solvent and in the presence of a base. According to another preferred embodiment the reaction is carried out in a mixture of an alcohol, such as ethanol, and an aqueous sodium salt, such as sodium hydroxide.

In a particular embodiment the compound of formula (II) can be prepared by oxidation of the alcohol of formula (III). The reaction is carried out in the presence of oxidising agents for oxidising alcohols to aldehydes, well known to the person skilled in the art. The election of the most suitable reagent is a matter of routine experimentation for the skilled person. However, according to a preferred embodiment, the oxidation reaction is carried out in the presence of oxalyl chloride and DMSO, followed by treatment with a base, e.g. triethyl amine.

In a particular embodiment, the compound of formula (III) can be prepared by reduction of the carboxylate group of a compound of formula (IV). The reaction is carried out in the presence of reducing agents for reducing carboxylates to alcohols, well known to the person skilled in the art. The election of the most suitable reagent is a matter of routine experimentation for said person skilled. However, according to a preferred embodiment, the reduction is carried out in the presence of NaBH₄.

In a particular embodiment, the compound of formula (IV) can be prepared by aromatization of a compound of formula (V). A number of reagents carrying out this reaction are well known to the person skilled in the art. The election of the most suitable reagent is a matter of routine experimentation for the skilled person. However, according to a preferred embodiment, the aromatization is carried out by heating in the presence of POCl₃, so as to provide a compound of formula (IV) wherein R₁ is chlorine.

According to a preferred embodiment, such compound may be transformed in to a compound of formula (IV) wherein R₁ is -O-R⁴ or -S-R⁵, by reaction with a sodium salt of the corresponding alcoxide or thiolate of formula -OR⁴ or -SR⁵, respectively. Alternatively, said transformation can be performed over a compound of formula (III), (II) or (I).

According to a further embodiment, the aromatization may take place in the presence of a base and an alkyl halide of formula R⁴X, wherein X is halogen. The election of the most suitable reagents is a matter of routine experimentation for the skilled person. However, according to a preferred embodiment, the base is sodium hydride and the alkyl halide is an alkyl iodide.

In a particular embodiment, the compound of formula (V) can be prepared by cyclation of a compound of formula (VI), under heat. In a further embodiment, the compound of formula (VI) can be prepared by alkylation of an amine of formula (VII) in the presence of methyl acetylenedicarboxylate.

In a particular embodiment, the compound of formula (VII) can be prepared by reduction of the nitro group of a compound of formula (VIII). The reaction is carried out in the presence of reducing agents for reducing nitro groups to amines, well known to the person skilled in the art. The election of the most suitable reagent is a matter of routine experimentation for said person skilled. However, according to a preferred embodiment, the reduction is carried out in the presence of tinchloride dihydrate.

In a particular embodiment, the compound of formula (VIII) can be prepared by condensation of a compound of formula (IX) with glycerol in acid media.

### EXAMPLES

In the present examples, the following compounds of formula (I) are being referred to:

**TABLE 1**

| **Compound** | **Structure** |
|---|---|
| **A** | 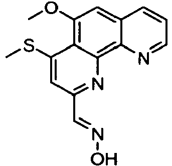 |
| **B** | 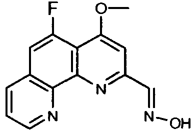 |
| **C** | 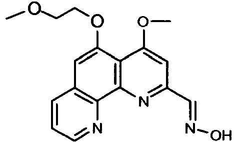 |
| **D** | 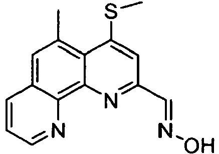 |
| **E** | 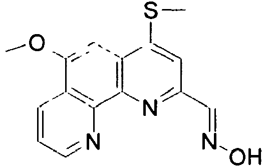 |
| **F** | 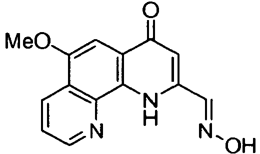 |

### SYNTHESIS OF THE COMPOUNDS

Compounds of formula (I) according to the present invention were prepared following the general preparation strategy detailed below.

In the following, the particular syntheses of compounds A to F, with structures as detailed in table 1, are described. The intermediate compounds are named with arabic figures.
All the reactives used are commercially available.

### Example 1

### Synthesis of Phenanthroline Derivative B

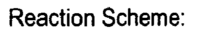

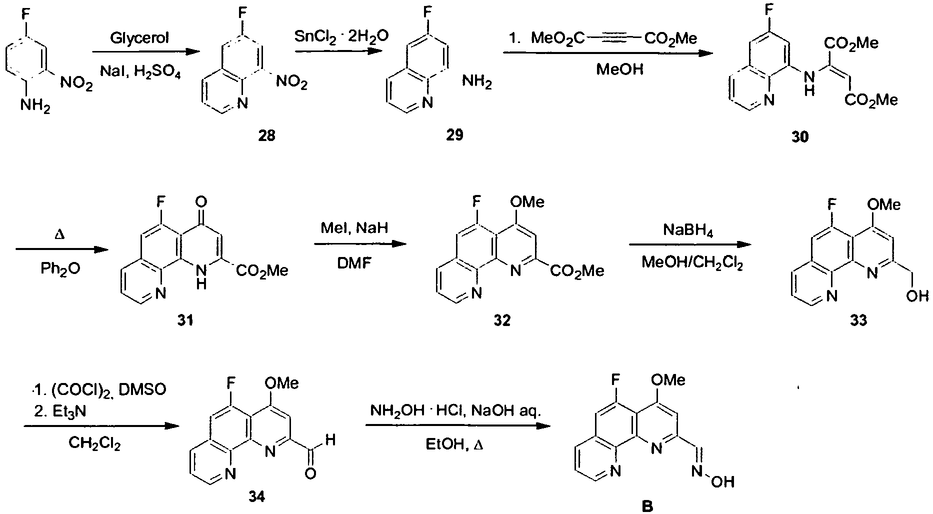

### 1. Synthesis of 6-fluoro-8-nitro-quinoline (28)

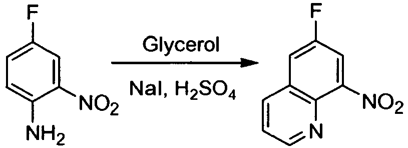

Glycerol (38.0 mL, 518.5 mmol) was heated at 140-150 °C for 1 hour. 4-Fluoro-2-nitrophenylamine (30.0 g, 192 mmol) and Nal (0.9 g, 5.8 mmol) were added and the mixture was cooled to 110 °C while H₂SO₄ (23.5 mL, 441.6 mmol) was slowly added from an addition funnel. The temperature was risen again to 150 °C and the reaction was stirred for 3 hours. The reaction was cooled down to room temperature and water and CH₂Cl₂ were added. The organic layer was dried (Na₂SO₄) and evaporated to yield the nitroquinoline 28 (14.9 g, 40%) as a yellow-brown solid, which was used in the next step without further purification.

¹H-NMR (400 MHz, CDCl₃, ppm): 9.05 (dd, 1 H, J= 1.76, 4.24 Hz); 8.23 (dd, 1H, J=1.65, 8.43 Hz); 7.86 (dd, 1H, J=2. 75, 7.95 Hz); 7.70 (dd, 1H, J=2.78, 8.07 Hz); 7.58 (dd, 1H, J=4.35, 8.68 Hz).

### 2. Synthesis of 6-fluoro-8-amino-quinoline (29)

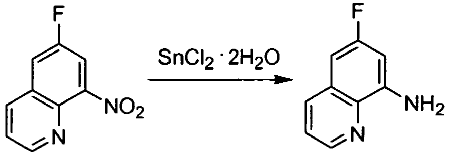

To a solution of the nitroquinoline 28 (14.9 g, 77.9 mmol) in ethanol (100 mL) SnCl₂·2H₂O (53.0 g, 233.6 mmol) was added. The reaction was heated to reflux for 2 hours. After cooling to room temperature, the reaction mixture was adjusted to pH 10 by the addition of 1N NaOH. The tin salts were filtered and washed 4 times with CH₂Cl₂. The combined organic liquors were washed with water, dried (Na₂SO₄) and evaporated to yield the aminoquinoline 29 (10.6 g, 84%) as a brown oil.

¹H-NMR (400 MHz, CDCl₃, ppm): 8.68 (dd, 1H, J=1.56, 4.16 Hz); 7.99 (dd, 1H, J=1.65, 8.23 Hz); 7.37 (dd, 1H, J= 4.32, 8.23 Hz); 6.73 (dd, 1H, J= 2.6, 9.31 Hz); 6.66 (d, 1H, J= 2.61, 10.55 Hz).

### 3. Synthesis of the 2-(6-fluoro-quinotin-8-ylamine)-but-2-enadioic acid dimethyl ester (30)

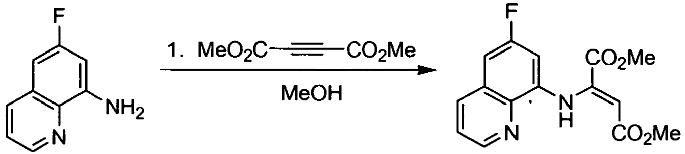

Methyl acetylenedicarboxylate (8.8 mL, 72.0 mmol) was added over a solution of the aminoquinoline 29 (10.6 g, 65.4 mmol) in MeOH (80 mL) at 0°C. The mixture was stirred at room temperature for 18 hours and a yellow precipitate appeared. The solid was filtered and washed with MeOH and hexane, affording the intermediate 30 (11.3 g, 57%) which was used in the next step without further purification.

¹H-NMR (400 MHz, CDCl₃, ppm): 11.07 (br, 1H); 8.86 (dd, 1 H, J= 1.6, 4.23 Hz); 8.07 (dd, 1H, J=1.60, 8.30 Hz); 7.46 (dd, 1H, J=4.23, 8.30 Hz); 7.04 (dd, 1H, J=2.50, 8.81 Hz); 6.65 (dd, 1H, J=2.48, 10.27 Hz); 5.66 (s, 1H); 3.83 (s, 3H); 3.80 (s, 3H).

### 4. Synthesis of 5-fluoro-4-oxo-1,4-dyhidro-[1,10]-phenanthroline-2-carboxylic acid methyl ester (31)

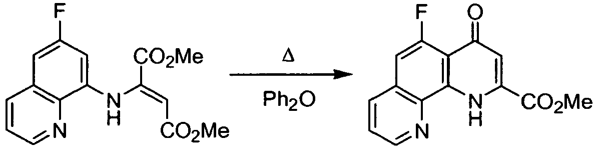

A solution of quinoline derivative 30 (6.7 g, 22.0 mmol) in diphenyl eter (20 mL) was slowly added (12 minutes) to refluxing diphenyl eter (100 mL). The reaction mixture was left to reflux for additional 10 minutes, and then left to reach room temperature. The product was precipitated by addition of hexanes (300 mL). The crude was filtered and washed with hexanes and ethyl ether. The obtained solid was treated with hot MeOH until compound 31 is totally eluted from a sintered funnel. After evaporation of the solvent, the brown solid obtained was washed with ethyl ether to afford the desired product 31 (2.4 g, 40%) as a brown solid.

¹H-NMR (400 MHz, CDCl₃, ppm): 8.89 (dd, 1H, J=1.43, 4.29 Hz); 8.16 (dd, 1H, J= 1.53, 8.28 Hz); 7.65 (dd, 1H, J= 4.31, 8.28 Hz); 7.24 (d, 1H, J=14.97 Hz); 7.16 (s, 1H); 4.99 (s, 3H).

### 5. Synthesis of 5-fluoro-4-methoxy-[1,10]phenanthroline-2-carboxylic acid methyl ester (32)

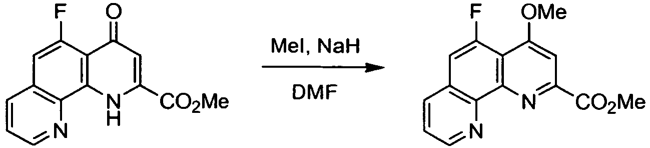

To a solution of the phenanthrolone 31 (500 mg, 1.83 mmol) and NaH (60 % in mineral oil, 5.50 mmol, 220 mg) in DMF (20 mL), methyl iodide (0.45 mL, 7.32 mmol) was added. The reaction was stirred at room temperature for 18 hours. Solvent was removed under reduced pressure and the crude was distributed in water and CH₂Cl₂. The organic layer was dried (Na₂SO₄) and evaporated under reduced pressure. The solid obtained was washed with ether to isolate the phenanthroline 32 (286 mg, 54%), which was used in the next step without further purification.

¹H-NMR (400 MHz, CDCl₃, ppm): 9.23 (dd, 1H, J=1.19, 4.18 Hz); 8.09 (dd, 1H, J= 1.63, 8.11 Hz); 7.94 (s, 1H); 7.67 (dd, 1H, J= 4.37, 8.08 Hz); 7.47 (d, 1H, J=12.23 Hz); 4.17 (s, 3H); 4.10 (s, 3H).

### 6. Synthesis of 5-fluoro-4-methoxy-[1,10]-phenanthrolin-2-yl]-methanol (33)

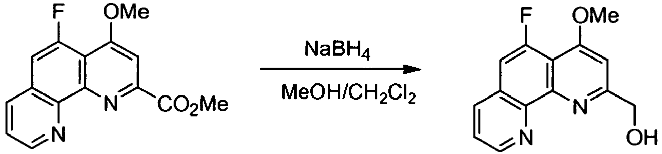

To a solution of ester 32 (445 mg, 1.56 mmol) in a mixture of MeOH/CH₂Cl₂ (1:5, 40 mL) at 0°C, solid NaBH₄ (72 mg, 1.87 mmol) was slowly added. The reaction was left to reach room temperature and stirred for 1 hour. The reaction mixture was poured into water, and extracted 3 times with CH₂Cl₂. The combined organic layers were dried (Na₂SO₄), and evaporated to afford alcohol 33 (346 mg, 77%) as a brown solid, which was used in the next step without further purification.

¹H-NMR (400 MHz, CDCl₃, ppm): 9.13 (dd, 1H); 8.23 (dd, 1H, J= 1.58, 8.14 Hz); 7.67 (dd, 1H, J=4.34, 7.73 Hz); 7.38 (d, 1H, J=12.3 Hz); 7.23 (s, 1H); 5.14 (s, 2H); 4.14 (s, 3H).

### 7. Synthesis of 5-fluoro-4-methoxy-[1,10]-phenanthrolin-2-carbaldehyde (34)

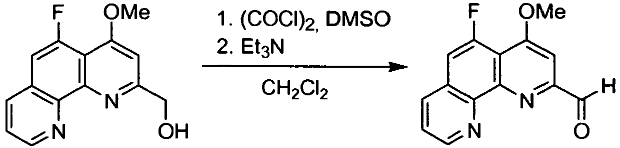

To a 2.0 M solution of oxalyl chloride in dry CH₂Cl₂ (1.34 mL, 2.68 mmol), a solution of dimethylsulfoxide in dry CH₂Cl₂ (0.38 mL, 5.36 mmol) was slowly added under nitrogen at - 78°C. The mixture was stirred for 35 minutes and a solution of 5-fluoro-4-methoxy-[1,10]-phenanthrolin-2-yl]-methanol 33 (346 mg, 1.34 mmol) in anhydrous CH₂Cl₂ (10mL) was added. After stirring for 2 hours at -78°C triethylamine (1.11 mL, 8.04 mmol) was added and the mixture was stirred at room temperature for 2 hours. The reaction mixture was washed with water, brine, dried (Na₂SO₄) and evaporated to obtain the desired aldehyde 34 (320 mg, 93%) as a red-brown solid.

¹H-NMR (400 MHz, CDCl₃, ppm): 10.48 (s, 1H); 9.23 (dd, 1H, J=1.99, 4.73 Hz); 8.23 (dd, 1H, J=1.70, 8.10 Hz); 7.75 (s, 1H); 7.70 (dd, 1H, J= 4.30, 8.15 Hz); 7.52 (d, 1H, J=12.26 Hz); 4.19 (s, 3H).

### 8. Synthesis of 5-fluoro-4-methoxy-[1,10]-phenanthroline-2-carbaldehyde oxime (B)

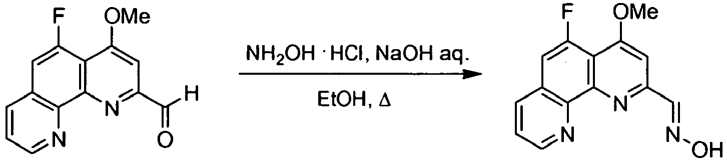

To a solution of the aldehyde derivative 34 (320 mg, 1.25 mmol) in ethanol (50 mL), a solution of hydroxylamine hydrochloride (434 mg, 6.25 mmol) in water (40 mL) was added and the mixture was heated at 35°C. A solution of 10% NaOH was added until pH 6, stirred at 35°C for 1 hour and cooled at room temperature. Water was added and the precipitate was filtered, washed with water and dried to give the aldoxime B (152 mg, 45%) as a white solid.

¹H-NMR (400 MHz, DMSO-d₆, ppm): 12.01 (s, 1H); 9.06 (dd, 1H, J=1.72, 4.25 Hz); 8.42 (dd, 1H, J=1.69, 8.14 Hz); 8.33 (s, 1H); 7.78 (dd, 1H, J= 4.27, 8.16 Hz); 7.74 (d, 1H, J=13.09 Hz); 7.66 (s, 1H); 3.32 (s, 3H).

### Example 2

### Synthesis of Phenanthroline Derivative C

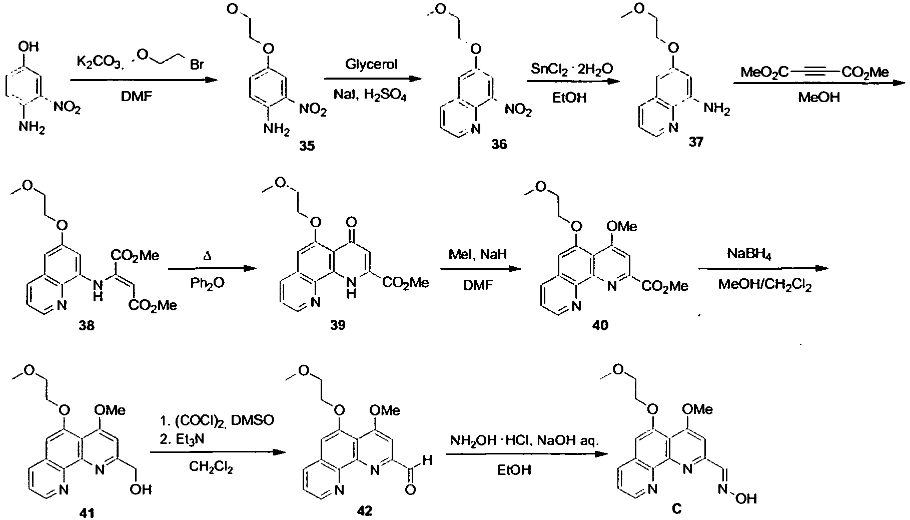

### 1. Synthesis of 4-(2-methoxy-ethoxy)-2-nitro-phenylamine (35)

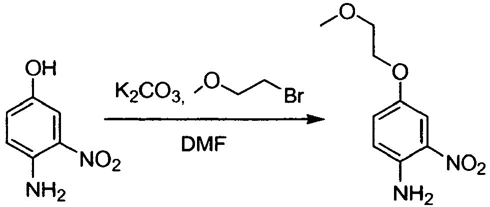

To a solution of 4-amine-3-nitrophenol (30.0 g, 195 mmol) and K₂CO₃ (53.8 g, 390 mmol) in DMF, 1-bromo-2-methoxy-ethane was added (20.0 mL, 214 mmol). The reaction was heated to 50 °C for 4 hours. The solvent was removed under reduced pressure and the residue was distributed in water and CH₂Cl₂. The organic layer was dried (Na₂SO₄) and evaporated to obtain the desired pure product 35 (33.8 g, 81%) as an orange solid.

¹H-NMR (400 MHz, CDCl₃, ppm): 7.99 (s, NH₂); 7.52 (d, 1H, J=2.92 Hz); 7.09 (dd, 1H, J= 6.12, 9.08 Hz); 6.76 (d, 1H, J= 9.15 Hz); 4.06 (m, 2H); 3.71 (m, 2H); 3.42(s, 3H).

### 2. Synthesis of 6-(2-methoxy-ethoxy)-8-nitro-quinotine (36)

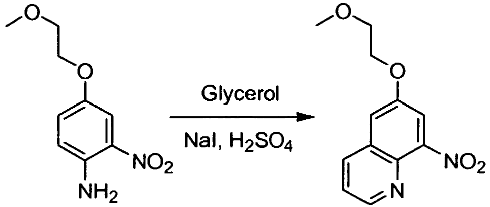

Glycerol (31.5 mL, 431.0 mmol) was heated at 140-150 °C for 1 hour and 4-(2-methoxy-ethoxy)-2-nitro-phenylamine 35 (33.38 g, 159.6 mmol) and Nal (0.48 g, 3. 2 mmol) were added. The reaction mixture was cooled to 110 °C and H₂SO₄ (19.6 mL, 367.0 mmol) was added dropwise from an addition funnel. The reaction was heated to 150 °C for 3 hours, cooled to room temperature and transferred to a separatory funnel with water and CH₂Cl₂. The organic layer was dried (Na₂SO₄) and evaporated. The residue obtained was purified by flash chromatography (SiO₂, ethyl acetate/hexane 1:1) yieding pure quinoline 36 (2.21 g. 6%) as a brown oil.

¹H-NMR (400 MHz, CDCl₃, ppm): 8.87 (dd, 1H, J= 1.62, 4.26 Hz); 8.10 (dd, 1H, J=1.63, 8.4 Hz); 7.73 (d, 1H, J=2.71 Hz); 7.45 (dd, 1H, J=4.24, 8.4 Hz); 7.29 (d, 1H, J= 2.72 Hz); 4.27 (m, 2H); 3.81 (m, 2H); 3.46 (s, 3H).

### 3. Synthesis of 6-(2-methoxy-ethoxy)-8-amino-quinoline (37)

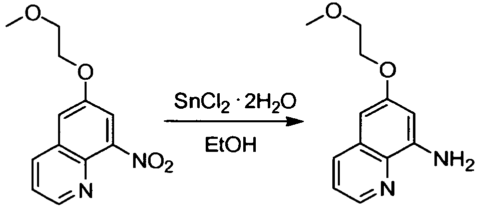

To a solution of nitroquinoline 36 (2.21 g, 8.9 mmol) in ethanol (20 mL) SnCl₂·2H₂O (6.00 g, 26.8 mmol) was added. The reaction was refluxed for 2 hours, cooled down to room temperature and adjusted to pH 10 by the addition of 1N NaOH. The tin salts were filtered off and rinsed with CH₂Cl₂. The combined organic liquors were washed with water, dried (Na₂SO₄) and evaporated to yield the aminoquinoline 37 (1.50 g, 78%) as a brown oil, which was used in the next step without further purification.

¹H-NMR (400 MHz, CDCl₃, ppm): 8.58 (dd, 1H, J=2.58, 4.22 Hz); 7.93 (dd, 1H, J=1.6, 8.3 Hz); 7.31 (dd, 1H, J= 4.21, 8.28 Hz); 6.63 (d, 1H, J= 2.55 Hz); 6.47 (d, 1H, J= 2.57 Hz); 4.19 (m, 2H); 3.79 (m, 2H); 3.47 (s, 3H).

### 4. Synthesis of 2-[6-(2-methoxy-ethoxy)quinolin-8-ylamine)-but-2-enadioic acid dimethyl ester (38)

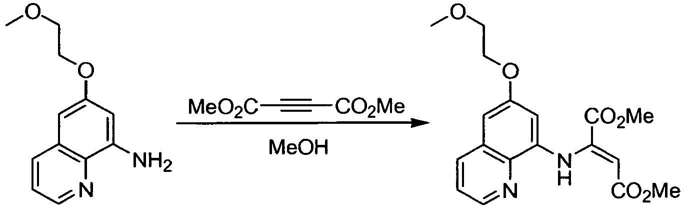

Methyl acetylenedicarboxylate (0.94 mL, 7.7 mmol) was added over a solution of the aminoquinoline 37 (1.52 g, 7.0 mmol) in MeOH (40 mL) at 0°C. The mixture was stirred at room temperature for 18 hours and a yellow precipitate appeared. The solid was filtered and washed with MeOH and hexane, affording the intermediate 38 (1.41 g, 51%), which was used in the next step without further purification.

¹H-NMR (400 MHz, CDCl₃, ppm): 10.91 (br, 1H); 8.75 (dd, 1H, J= 1.60, 4.16 Hz); 7.99 (dd, 1H, J= 1.60, 8.29 Hz); 7.38 (dd, 1H, J= 4.25, 8.27 Hz); 6.74 (d, 1H, J=2.45 Hz); 6.64 (d, 1H, J= 2.47 Hz); 5.58 (s, 1H); 4.20 (m, 2H); 3.79 (m, 8H), 3.46 (s, 3H).

### 5. Synthesis of 5-(2-methoxy-ethoxy)-4-oxo-1,4-dyhidro-[1,10]-phenanthrotine-2-carboxylic acid methyl ester (39)

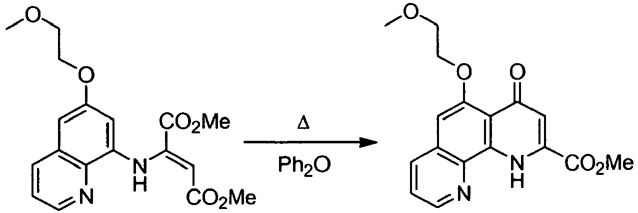

A solution of quinoline derivative 38 (1.41 g, 3.6 mmol) in diphenyl ether (10 mL) was slowly added (12 minutes) to refluxing diphenyl ether (60 mL). The reaction mixture was left to reflux for additional 10 minutes, and then left to reach room temperature. The product was precipitated by the addition of hexane. The residue was filtered and washed with hexane and ethyl ether. The obtained solid was treated with hot MeOH until compound 39 was totally eluted from a sintered funnel. After solvent evaporation, the brown solid obtained was washed with ethyl ether to afford the desired product 39 (0.70 g, 60%) as a dark brown solid.

¹H-NMR (400 MHz, CDCl₃, ppm): 8.79 (dd, 1H); 8.06 (dd, 1H, J= 1.54, 8.24 Hz); 7.55 (dd, 1H, J= 4.31, 8.23 Hz); 7.24 (d, 1H, J=14.96 Hz); 6.86 (s, 1H); 4.33 (m, 2H); 4.06 (s, 3H); 3.96 (m, 2H); 3.56 (s, 3H).

### 6. Synthesis of 4-methoxy-5-(2-methoxy-ethoxy)-[1,10]-phenanthroline-2-carboxylic acid methyl ester (40)

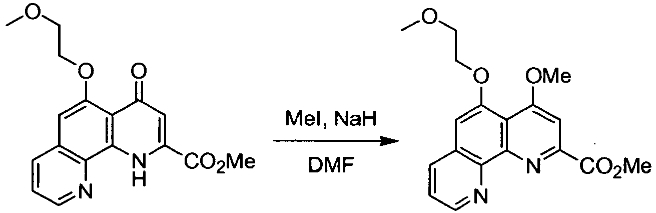

To a solution of the phenanthrolone 39 (703 mg, 2.15 mmol) and NaH (60 % in mineral oil, 258 mg, 6.45 mmol) in DMF (20 mL), methyl iodide (0.45 mL, 7.32 mmol) was added. The reaction was stirred at room temperature for 18 hours. Solvent was removed under reduced pressure and the crude was distributed in water and CH₂Cl₂. The organic layer was dried (Na₂SO₄) and evaporated under reduced pressure. The solid obtained was washed with ether to isolate the phenanthroline 40 (272 mg, 37%) as a brown oil, which was used in the next step without further purification.

¹H-NMR (400 MHz, CDCl₃, ppm): 9.07 (dd, 1H); 8.09 (dd, 1H, J= 1.44, 8.08 Hz); 7.87 (s, 1H); 7.57 (dd, 1H, J= 4.37, 8.08 Hz); 7.06 (s, 1H); 4.34 (m, 2H); 4.13 (s, 3H); 4.09 (s, 3H); 3.96 (m, 2H); 3.55 (s, 3H).

### 7. Synthesis of [4-methoxy-5-(2-methoxy-ethoxy)-[1,10]-phenanthrolin-2-yl]-methanol (41)

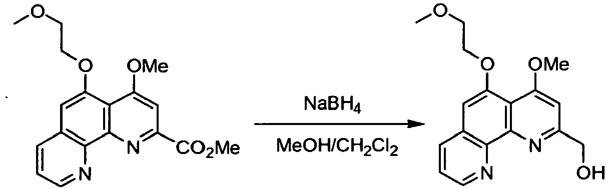

To a solution of ester 40 (272 mg, 0.79 mmol) in a mixture of MeOH/CH₂Cl₂ (1:5, 40 mL) at 0°C, solid NaBH₄ (36 mg, 0.95 mmol) was slowly added. The reaction was left to reach room temperature and stirred for 1 hour. The reaction mixture was poured into water, and extracted 3 times with CH₂Cl₂. The combined organic layers were dried (Na₂SO₄), and evaporated to afford alcohol 41 (210 mg, 84%) as a brown oil, which was used in the next step without further purification.

¹H-NMR (400 MHz, CDCl₃, ppm): 8.97 (dd, 1H); 8.08 (dd, 1H); 7.87 (s, 1H); 7.53 (dd, 1H); 7.16 (s, 1H); 6.96 (s, 1H); 5.10 (s, 2H); 4.34 (m, 2H); 4.06 (s, 3H); 3.96 (m, 2H); 3.56 (s, 3H)

### 8. Synthesis of 4-methoxy-5-(2-methoxy-ethoxy)-[1,10]phenanthrotine-2-carbaldehyde (42)

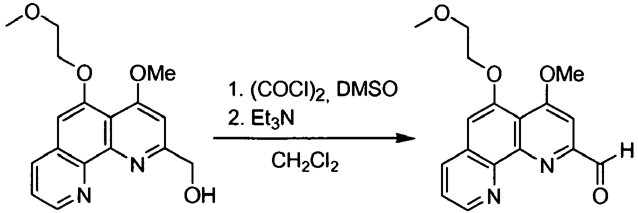

To a 2.0 M solution of oxalyl chloride in dry CH₂Cl₂ (0.67 mL, 1.34 mmol) a solution of dimethylsulfoxide in dry CH₂Cl₂ (0.19 mL, 2.68 mmol) was slowly added under nitrogen at - 78°C. The mixture was stirred for 35 minutes and a solution of [4-methoxy-5-(2-methoxyethoxy)[1,10]-phenanthrolin-2-yl]methanol 41 (210 mg, 0.67 mmol) in anhydrous CH₂Cl₂ (10mL) was added. After stirring for 2 hours at -78°C, triethylamine (0.56 mL, 4.02 mmol) was added and the mixture was stirred under nitrogen at room temperature for 2 hours. The reaction mixture was washed with water, brine, dried (Na₂SO₄) and evaporated to obtain the desired aldehyde 42 (198 mg, 97%) as a brown solid, which was used in the next step without further purification.

¹H-NMR (400 MHz, CDCl₃, ppm): 10.45 (s, 1H); 9.08 (dd, 1H, J=1.46, 4.27 Hz); 8.09 (dd, 1H, J=1.34, 8.12 Hz); 7.67 (s, 1H); 7.58 (dd, 1H, J= 4.32, 8.04 Hz); 7.08 (s, 1H); 4.32 (m, 2H); 4.11 (s, 3H); 3.91 (m, 2H); 3.53 (s, 3H).

### 9. Synthesis of 4-methoxy-5-(2-methoxy-ethoxy)-[1,10]-phenanthroline-2-carbaldehyde oxime (C)

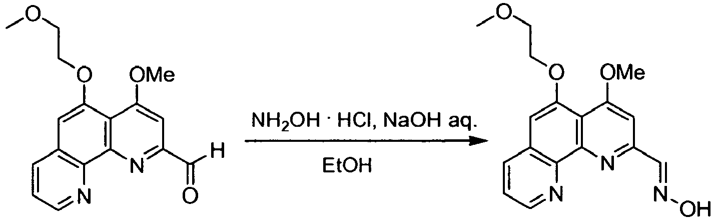

To a solution of the aldehyde derivative 42 (198 mg, 0.65 mmol) in ethanol (40 mL), a solution of hydroxylamine hydrochloride (225 mg, 3.25 mmol) in water (40 mL) was added . A solution of 10% NaOH was added until pH 6, and the mixture was stirred at room temperature for 1 hour. Water was added and the reaction was extracted with CH₂Cl₂. The organic layer was dried (Na₂SO₄) and evaporated under reduced pressure yielding the desired aldoxime C (69 mg, 32%) as a white solid.

¹H-NMR (400 MHz, DMSO-d₆, ppm): 11.95 (s, 1H); 8.90 (dd, 1H, J=1.72, 4.22 Hz); 8.30 (s, 1H); 8.27 (dd, 1H, J=1.72, 8.21 Hz); 7.67 (dd, 1H, J= 4.27, 8.11 Hz); 7.58 (s, 1H); 7.30 (s, 1H); 4.29 (m, 2H); 4.03 (s, 3H); 3.84 (m, 2H); 3.43 (s, 3H).

### Example 3

### Synthesis of Phenanthroline Derivative E

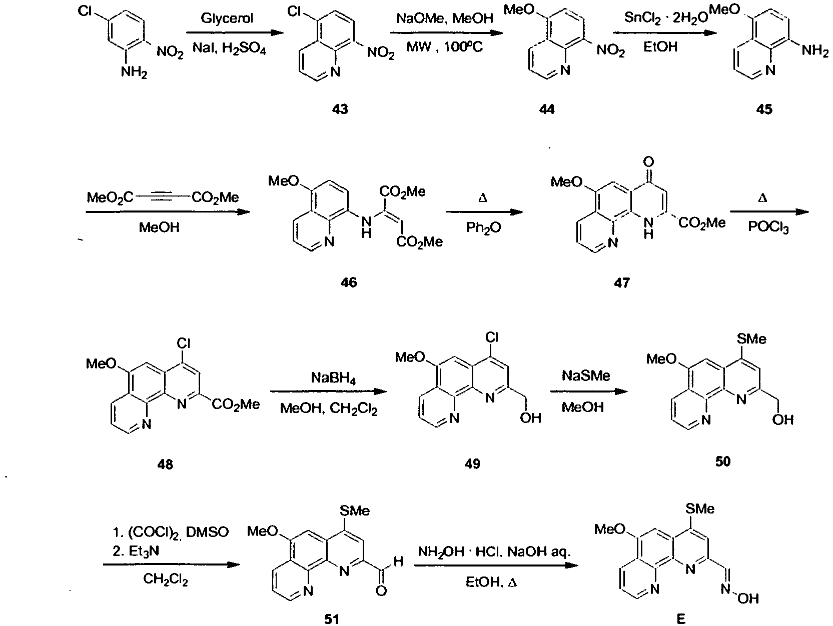

### 1. Synthesis of 5-chloro-8-nitro-quinoline (43)

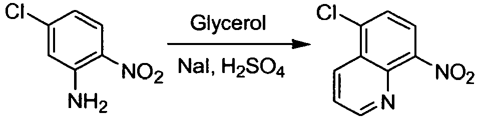

To glycerol (11.4 ml, 156.5 mmol) preheated to 160°C for 1 hour and cooled down to 110°C, 5-chloro-2-nitroaniline (10.00 g, 58.0 mmol) and sodium iodide (0.17g, 1.2 mmol) were added. The mixture was vigorously stirred at 110°C to get a homogeneus tar. The reaction mixture was heated again to 150°C, and sulfuric acid 95-98% (7.1 mL, 133.3 mmol) was added dropwise. After 45 minutes stirring at 150°C, the reaction was allowed to reach room temperature, and was distributed in CH₂Cl₂ and 50% H₂SO₄. The organic layer was dried (Na₂SO₄) and evaporated to dryness. The residue was washed with MeOH/ hexane (1:5), yielding the desired product 43 (7.33 g, 61%) as an orange solid.

¹H-NMR (400 MHz, CDCl₃, ppm): 9.12 (H-2, dd, 1H, J=1.6 and 4.2 Hz), 8.67 (H-4, dd, 1H, J=1.6 and 8.6 Hz), 7.99 (H-7, d, 1H, J=8.1 Hz), 7.71 (H-6, d, 1H, J=8.1 Hz), 7.68 (H-3, dd, 1H, J=4.2 and 8.6 Hz).

### 2. Synthesis of 5-methoxy-8-nitro-quinoline (44)

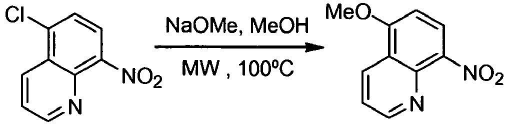

A mixture of sodium methoxide (7.3 g, 135.7 mmol) and 5-chloro-8-nitroquinoline 43 (6.7 g , 32.2 mmol), in anhydrous MeOH (80 ml), was heated in a microwave oven (power: 250W, ramp time: 5 minutes) at 100°C for 10 minutes. The solvent was partially evaporated and CH₂Cl₂ and water were added. The organic layer was collected and washed with water and brine, dried (Na₂SO₄) and evaporated. The residue was treated with MeOH/hexane (3:50) to afford 5-methoxy-8-nitroquinoline 44 (5.8 g, 83%) as a brown solid.

¹H-NMR (400 MHz, CDCl₃, ppm): 9.08 (H-2, dd, 1H, J=1.8 and 4.2 Hz), 8.63 (H-4, dd, 1H, J=1.8 and 8.6 Hz), 8.19 (H-7, d, 1H, J=8.6 Hz), 7.51 (H-3, dd, 1H, J=4.2 and 8.6 Hz), 6.84 (H-6, d, 1H, J=8.6 Hz), 4.09 (OCH₃, s, 3H).

### 3. Synthesis of 5-methoxy-quinolin-8-ylamine (45)

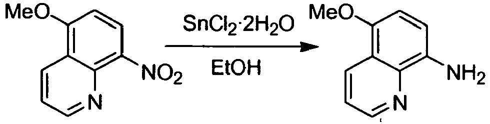

A suspension of 5-methoxy-8-nitroquinoline 44 (5.8 g, 28.5 mmol) and SnCl₂·2H₂O (19.3 g, 85.6 mmol) in ethanol (120 mL) was heated to reflux for 1 hour. The reaction mixture was diluted with ethyl acetate and treated with 10% NaOH up to pH 11. The organic layer was separated, washed with brine, dried (Na₂SO₄), filtered and evaporated. The residue was purified by flash chromatography (SiO₂, ethyl acetate/hexane, 0-40%) to obtain the final amine 45 (2.7 g, 53%) as a brown solid.

¹H-NMR (400 MHz, CDCl₃, ppm): 8.79 (H-2, dd, 1H, J=1.7 and 4.2 Hz), 8.51 (H-4, dd, 1H, J=1.7 and 8.5 Hz), 7.38 (H-3, dd, 1H, J=4.2 and 8.5 Hz), 6.89 (H-7, d, 1H, J=8.2 Hz), 6.73 (H-6, d, 1H, J=8.2 Hz), 3.95 (OCH₃, s, 3H).

### 4. Synthesis of 2-(5-methoxyquinolin-8-ylamino)-but-2-enedioic acid dimethyl ester (46)

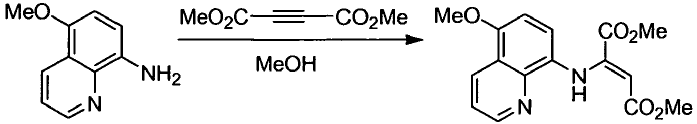

To a solution of 5-methoxyquinolin-8-ylamine 45 (2.7 g, 15.3 mmol) in methanol (20 mL) at 4°C, dimethylacetylenedicarboxylate (2.1 mL, 16.8 mmol) was added. The reaction mixture was stirred and allowed to warm to room temperature for 3 hours. The solvent was evaporated and the residue was washed with MeOH and hexane, yielding an orange solid that was dried under vacuum (3.8 g, 79%).

¹H-NMR (400 MHz, CDCl₃, ppm): 10.63 (NH, s, 1H), 8.90 (H-2, dd, 1H, J=1.7 and 4.2 Hz), 8.53 (H-4, dd, 1H, J=1.7 and 8.4 Hz), 7.41 (H-3, dd, 1H, J=4.2 and 8.4 Hz), 6.95 (H-7, d, 1H, J=8.3 Hz), 6.73 (H-6, d, 1H, J=8.3Hz), 5.44 (s, 1H, CH=), 3.96 (OCH3, s, 3H), 3.77 (CO₂CH₃, s, 3H), 3.69 (CO₂CH₃, s, 3H).

### 5. Synthesis of 6-methoxy-4-oxo-1,4-dihydro-[1,10]phenanthroline-2-carboxylic acid methyl ester (47)

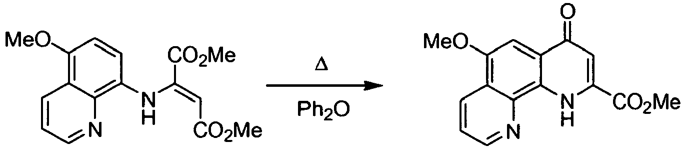

A solution of 2-(5-methoxyquinolin-8-ylamino}-but-2-enedioic acid dimethyl ester 46 (3.8 g, 12.1 mmol) in diphenyl ether (25 mL) was refluxed at 250°C for 15 minutes. The reaction mixture was allowed to warm to room temperature, and then hexane was added to afford a brown solid (3.0 g, 88%), that was filtered and dried under vacuum.

¹H-NMR (400 MHz, CDCl₃, ppm): 10.92 (NH, s, 1H), 8.97 (H-9, dd, 1H, J=1.6 and 4.3 Hz), 8.66 (H-7, dd, 1H, J=1.6 and 8.5 Hz), 7.66 (H-8, dd, 1H, J=4.3 and 8.5 Hz), 7.59 (H-3, s, 1H), 7.26 (H-5, s, 1H), 4.11 (OCH₃, s, 3H), 4.08 (OCH₃, s, 3H).

### 6. Synthesis of 4-chloro-6-methoxy-[1,10]phenanthroline-2-carboxylic acid methyl ester (48)

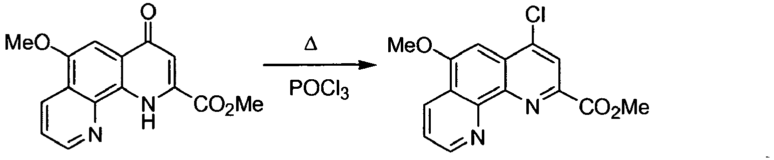

To solid 6-methoxy-4-oxo-1,4-dihydro-[1,10]phenanthroline-2-carboxylic acid methyl ester 47 (2.01 g, 7.07 mmol), POCl₃ (18 mL) was slowly added and the mixture was heated to reflux for 2 hours. The reaction was cooled down to room temperature and the solvent was evaporated. The solid obtained was treated with CH₂Cl₂ and saturated NaHCO₃ and transferred to a separatory funnel. The aqueous layer was further extracted with CH₂Cl₂ and the combined organic layers were washed with brine, dried (Na₂SO₄), filtered and evaporated. The residue obtained was treated with ethyl ether, filtered and dried affording the chloro derivative 48 as a light brown solid (2.02 g, 95%).

¹H-NMR (400 MHz, CDCl₃, ppm): 9.89 (d, 1H, J=5.0 Hz), 9.19 (d, 1H, J=8.3 Hz), 8.54 (s, 1H), 8.16 (dd, 1H, J=5.0 and 8.3Hz), 7.55 (s, 1H), 4.27 (s, 3H), 4.15 (s, 3H).

### 7. Synthesis of (4-chloro-6-methoxy-[1,10]phenanthrolin-2-yl)-methanol (49)

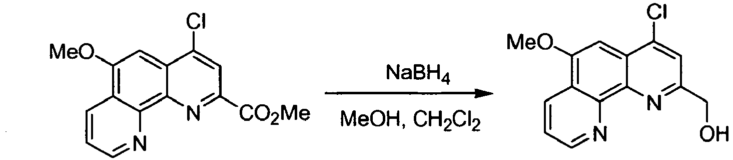

To a solution of 4-chloro-6-methoxy-[1,10]phenanthroline-2-carboxylic acid methyl ester 48 (2.02 g, 6.63 mmol) in a mixture MeOH/CH₂Cl₂ (1:5, 300 mL) cooled at 0°C, solid NaBH₄ ( 0.71 g, 19.80 mmol) was added. The mixture was stirred at 0°C for 3 hours and at room temperature for 18 hours. The reaction mixture was diluted with water and the organic layer was separated. The aqueous layer was extracted twice with CH₂Cl₂, and the combined organic layers were dried (Na₂SO₄), evaporated and the residue was washed with ethyl ether to afford the alcohol 49 (1.72 g, 94%) as a light brown solid.

¹H-NMR (400 MHz, CDCl₃, ppm): 9.17 (d, 1H, J=2.6 Hz), 8.70 (dd, 1H, J=1.7Hz and 8.2 Hz), 7.78 (s, 1H), 7.66 (dd, 1H, J=4.4and 8.2 Hz), 7.36 (s, 1H), 5.09 (s, 2H), 4.16 (s, 3H).

### 8. Synthesis of (6-methoxy-4-methylsulfanyl-(1,10]phenanthrolin-2-yl)-methanol (50)

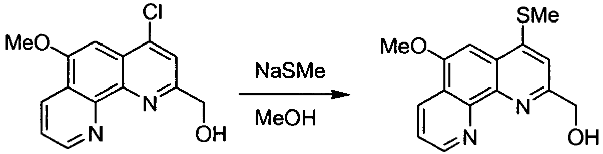

Solid sodium thiomethylate (2.18 g, 31.0 mmol) was added to a solution of 4-chloro-6-methoxy-[1,10]phenanthrolin-2-yl)-methanol 49 (1.72 g, 6.21 mmol) in MeOH (300 mL) and the reaction mixture was heated to reflux for 3 hours. Solvent was removed in a rotary evaporator and the residue was distributed in CH₂Cl₂ and saturated NaHCO₃. The organic layer was washed with brine, dried (Na₂SO₄), filtered and concentrated in vacuo. The solid residue was treated with ethyl ether, filtered, and dried to obtain the desired material 50 (0.74 g, 42%) as a light brown solid.

¹H-NMR (400 MHz, CDCl₃, ppm): 9.04 (s, 1H), 8.62 (d, 1H, J=6.8 Hz), 7.56 (s, 1H), 7.30 (m, 1H), 7.14 (s, 1H), 5.04 (s, 2H), 4.11 (s, 3H), 2.63 (s, 3H).

### 9. Synthesis of 6-methoxy-4-methylsulfanyl-[1,10]phenanthroline-2-carbaldehyde (51)

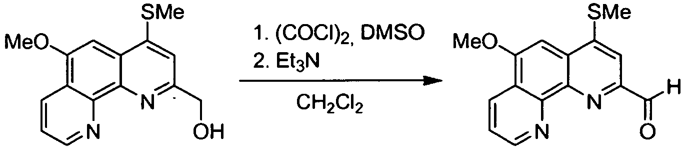

To a 2.0 M solution of oxalyl chloride in dry CH₂Cl₂ (2.56 mL, 5.12 mmol) at -78°C a solution of dimethylsulfoxide (0.73 mL, 10.24 mmol) in dry CH₂Cl₂ (15 mL) was slowly added under nitrogen. The mixture was stirred for 30 minutes and a solution of (6-methoxy-4-methylsulfanyl-[1,10]phenanthrolin-2-yl)-methanol 50 (738 mg, 2.58 mmol) in dry CH₂Cl₂ (20 mL) was added. After stirring at -78°C for 50 minutes, triethylamine (2.13 mL, 15.4 mmol) was added and the mixture was left to reach room temperature and stirred under nitrogen for 18 hours. The reaction mixture was washed with water, brine, dried (Na₂SO₄) and evaporated to obtain the aldehyde 51 (285 mg, 39 %) as a brown solid.

¹H-NMR (400 MHz, CDCl₃, ppm): 10.46 (s, 1H), 9.31 (dd, 1H, J=1.5 and 4.3 Hz), 8.75 (d, 1H, J=7.4 Hz), 8.01 (s, 1H), 7.74 (dd, 1H, J=4.3 and 8.2 Hz), 7.26 (s, 1H), 4.19 (s, 3H), 2.75 (s, 3H).

### 10. Synthesis of 6-methoxy-4-methylsulfanyl-[1,10]phenanthroline-2-carbaidehyde oxyme (E)

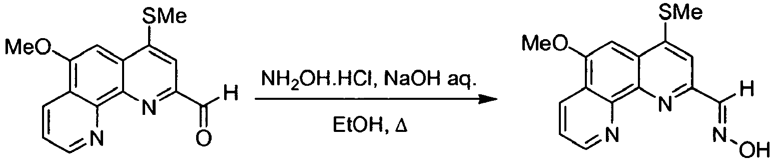

To a solution of 6-methoxy-4-methylsulfanyl-[1,10]phenanthroline-2-carbaldehyde 51 (285 mg, 1.10 mmol) in ethanol (15 mL), a solution of hydroxylamine hydrochloride (690 mg, 10.20 mmol) in water (20 mL) was added and the mixture was heated at 60°C. 10% NaOH was added until pH 6, and the reaction mixture was stirred at 60°C for 30 minutes and cooled to 0°C. The resulting precipitate was filtered and washed successively with water and ethyl ether, to give the aldoxime E (120 mg, 40%) as a light yellow solid.

¹H-NMR (400 MHz, DMSO-d₆, ppm): 11.81 (s, 1H), 9.14 (dd, 1H, J=1.6 and 4.2 Hz), 8.64 (dd, 1H, J=1.7 and 8.3 Hz), 8.30 (s, 1H), 7.86 (s, 1H), 7.80 (dd, 1H, J=4.3 and 8.3Hz), 7.19 (s, 1H), 4.11 (s, 3H), 2.71 (s, 3H).

¹³C-RMN (400MHz, DMSO-d₆, ppm): 153.59, 151.59, 149.92, 149.91, 149.67, 147.33, 146.41, 141.034, 131.21, 126.73, 124.10, 123.43, 97.42, 56.79, 14.27.

MS (ES⁺): m/z = 302 (M+H)⁺

### Example 4

### Synthesis of Phenanthroline Derivative F

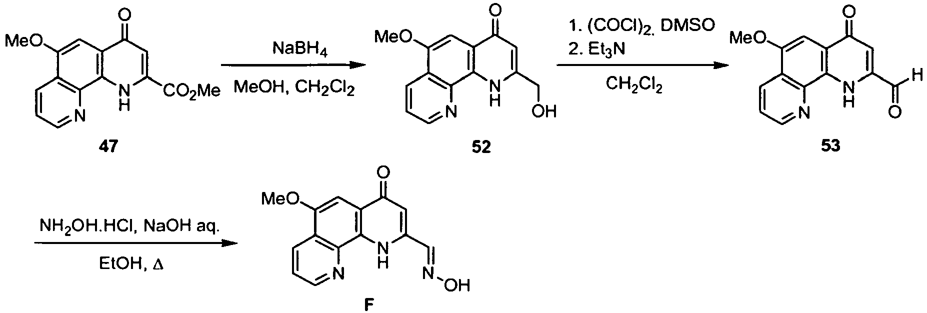

### 1. Synthesis of 2-hydroxymethyl-6-methoxy-1H-[1,10]phenanthrolin-4-one (52)

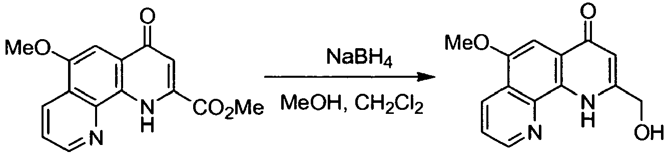

Solid NaBH₄ (1.20 g, 32.0 mmol) was added portionwise into a solution of 6-methoxy-4-oxo-1,4-dihydro-[1,10]phenanthroline-2-carboxylic acid methyl ester 47 (0.40 g, 1.4 mmol) in a mixture of MeOH/CH₂Cl₂ (1:1, 10 mL) cooled at 0°C. The mixture was stirred at 0°C for 3 hours, and then allowed to warm to room temperature and stirred for additional 24 hours. After dilution in MeOH/CH₂Cl₂ (1:3), the mixture was washed with water, and the organic layer was separated and evaporated till dryness. The residue was purified by flash chromatography (SiO₂, 7N NH₃OH in MeOH/CH₂Cl₂, 0-50%), yielding the desired alcohol 52 as a brown solid (0.25 g, 68%).

¹H-NMR (400 MHz, MeOD/CDCl₃, 1/1, ppm): 8.84 (H-9, dd, 1H, J=1.3 and 4.3 Hz), 8.43 (H-7, dd, 1H, J=1.3 and 8.5 Hz), 7.55 (H-8, dd, 1H, J=4.3 and 8.5 Hz), 7.28 (H-3, s, 1H), 6.32 (H-5, s, 1H), 4.77 (OCH₂, s, 2H), 3.98 (OCH₃, s, 3H).

### 2. Synthesis of 6-methoxy-4-oxo-1,4-dihydro-[1,10]phenanthroline-2-carbaldehyde (53)

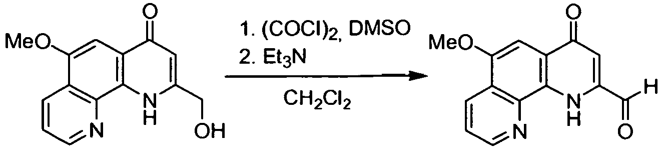

To a solution of 2.0 M of oxalyl chloride in anhydrous CH₂Cl₂ (270 µL, 0.54 mmol) at -78°C, a solution of anhydrous DMSO (77 µL, 1.08 mmol) in dry CH₂Cl₂ (1 mL) was added under nitrogen and stirred for 20 minutes. Then, a solution of 2-hydroxymethyl-6-methoxy-1H-[1,10]phenanthrolin-4-one 52 (68.5 mg, 0.27 mmol) in a mixture of anhydrous DMSO/CH₂Cl₂ (1:4, 2.5 mL) was added into the reaction mixture, and stirring was kept at -78°C for 50 additional minutes. Finally, triethylamine (225 µL, 1.6 mmol) was added at -78°C, and the reaction mixture was allowed to warm to room temperature for 3 hours. The final solution was diluted with CH₂Cl₂ and washed with water. The organic layer was separated, dried (Na₂SO₄), filtered and evaporated. The residue was purified by flash chromatography (SiO₂, MeOH/CH₂Cl₂, 0-50%), yielding a mixture that contained the aldehyde 53 which was used in the next step without further purification.

### 3. Synthesis of 6-methoxy-4-oxo-1,4-dihydro-[1,10]phenanthroline-2-carbaidehyde oxime (F)

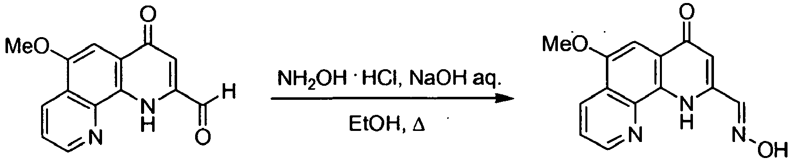

A solution of hydroxylamine hydrochloride (230 mg, 3.3 mmol) in water (1 ml) was added into a solution of 6-methoxy-4-oxo-1,4-dihydro-[1,10]phenanthroline-2-carbaldehyde (mixture obtained in the previous step) dissolved in ethanol/CH₂Cl₂ (3:1, 2 mL), and stirred at room temperature for 48 hours. The reaction mixture was diluted with CH₂Cl₂ and water, and the organic layer was separated, dried (Na₂SO₄), filtered and evaporated. The residue was purified by flash chromatography (SiO₂, 7N NH₃OH in MeOH/CH₂Cl₂, 0-50%) yielding an impure solid that was redissolved in MeOH/CHCl₃ and precipitated with ethyl ether to afford pure aldoxime F (6.2 mg, 4%).

¹H-NMR (400 MHz, MeOD/CDCl₃, 1/1, ppm): 9.02 (H-9, d, 1H, J= 4.0 Hz), 8.72 (H-7, d, 1H, J= 8.0 Hz), 8.19 (H-5, s, 1H), 7.77 (H-8, m, 1H), 7.52 (H-3, s, 1H), 6.68 (CH=, s, 1H), 4.12 (OCH₃, s, 3H).

MS (ES+): m/z = 270.

### Example 5

### Synthesis of Phenanthroline Derivative D

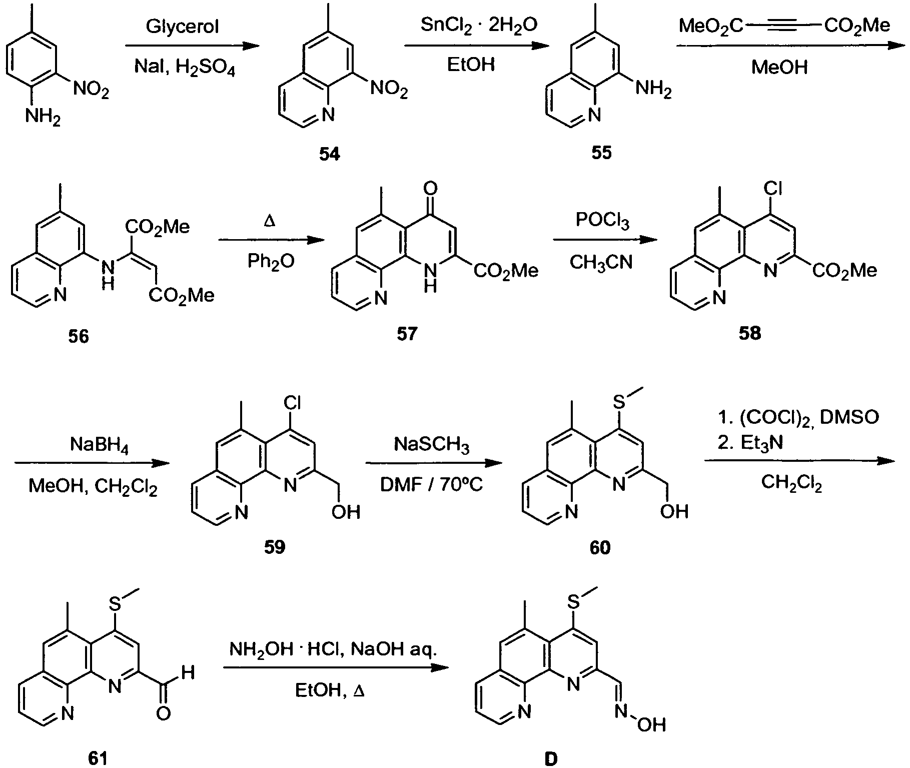

### 1. Synthesis of 6-methyl-8-nitroquinoline (54)

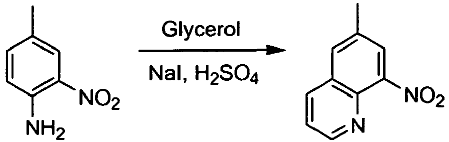

To glycerol (6.5 mL, 89.0 mmol) preheated to 160°C for 1 hour, and cooled down to 110°C, 4-methyl-2-nitroaniline (5.00 g, 33.0 mmol) and sodium iodide (0.10 g, 0.7 mmol) were added. The mixture was vigorously stirred, heated to 150 °C and sulfuric acid 95-98% (4.2 ml, 78.0 mmol) was added dropwise. After 45 minutes at 150°C, the mixture was allowed to reach room temperature, and then distributed in CH₂Cl₂ and water. The organic layer was dried (Na₂SO₄), filtered and evaporated. The residue was washed with MeOH/hexane (1:10), yielding the nitroquinoline **54** (4.07 g, 65%) as an orange solid that was dried under vacuum.

¹H-NMR (400 MHz, CDCl₃, ppm): 9.00 (H-2, dd, 1H, J=1.3 and 4.2 Hz), 8.16 (H-4, dd, 1H, J=1.3 and 8.3Hz), 7.89 (H-7, s, 1H), 7.80 (H-5, s, 1H), 7.50 (H-3, dd, 1H, J=4.2 and 8.3 Hz), 2.61 (CH₃, s, 3H).

### 2. Synthesis of 6-methyl-quinolin-8-ylamine (55)

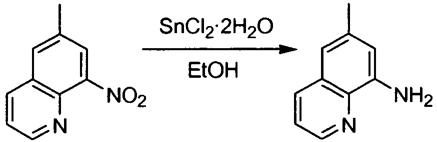

A suspension of 6-methyl-8-nitroquinoline (15.0 g, 80.0 mmol) and SnCl₂·2H₂O (54.0 g, 240.0 mmol) in ethanol (300 mL) was heated to reflux for 1 hour. The reaction mixture was diluted with ethyl acetate and treated with 10% KOH up to pH 11. The organic layer was separated, washed with brine, dried (Na₂SO₄), filtered and evaporated. The residue was purified by flash chromatography (SiO₂, ethyl acetate/hexane, 0-30%). The amine 55 (9.9 g, 79%) was isolated as a yellow solid.

¹H-NMR (400 MHz, CDCl₃, ppm): 8.69 (H-2, dd, 1H, J=1.6 and 4.1 Hz), 7.96 (H-4, dd, 1H, J=1.6 and 8.3 Hz), 7.33 (H-3, dd, 1H, J=4.1 and 8.3 Hz), 6.94 (H-5, s, 1H), 6.79 (H-7, s, 1H), 4.91 (NH₂, ws, 2H), 2.43 (CH₃, s, 3H).

### 3. Synthesis of 2-(6-methyl-quinolin-8-ylamino)-but-2-enedioic acid dimethyl ester (56)

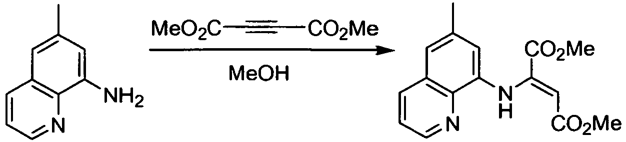

To a solution of 6-methyl-quinolin-8-ylamine **55** (4.8 g, 30.2 mmol) in MeOH (15 ml) at 4°C dimethylacetylenedicarboxylate (4.5 ml, 36.5 mmol) was added. The reaction mixture was stirred and allowed to warm to room temperature for 1 hour. The precipitate was filtered and washed thoroughly with MeOH and hexane to yield the desired compound **56** as a yellow solid (8.6 g, 95%).

¹H-NMR (400 MHz, CDCl₃, ppm): 10.89 (NH, s, 1H), 8.83 (H-2, dd, 1H, J=1.7 and 4.2 Hz), 8.02 (H-4, dd, 1H, J=1.7 and 8.3 Hz), 7.38 (H-3, dd, 1H, J=4.2 and 8.3 Hz), 7.23 (H-5, s, 1H), 6.79 (H-7, s, 1H), 5.54 (CH=, s, 1H), 3.80 (CO₂CH₃, s, 3H), 3.74 (CO₂CH₃, s, 3H), 2.46 (CH₃, s, 3H).

### 4. Synthesis of 5-methyl-4-oxo-1,4-dihydro-[1,10]phenanthroline-2-carboxylic acid methyl ester (57)

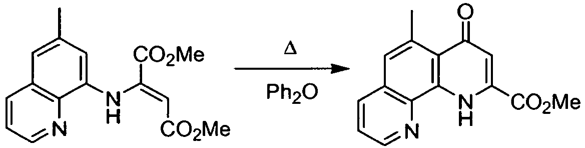

A solution of 2-(6-methylquinolin-8-ylamino)-but-2-enedioic acid dimethyl ester (8.6 g, 28.6 mmol) in diphenyl ether (50 mL) was distributed in ten reactors and each one was heated in a microwave oven (power: 250W, ramp time: 5 minutes) at 230°C for 10 minutes. The reaction mixtures were combined and filtered. After washing with MeOH and CH₂Cl₂, the filtrate was evaporated and a brown oil was obtained that precipitated upon addition of hexane. The solid was washed sequentally with hexane, MeOH and ethyl ether to yield the final compond **57** (5.7 g, 74%) as a brown solid.

¹H-NMR (400 MHz, CDCl₃, ppm): 11.07 (NH, s, 1H), 8.87 (H-9, d, 1H, J=4.3 Hz), 8.11 (H-7, d, 1H, J=8.1 Hz), 7.59 (H-8, dd, 1H, J=4.3 and 8.1 Hz), 7.31 (H-3, s, 1H), 7.10 (H-6, s, 1H), 4.07 (OCH₃, s, 3H), 3.01 (CH₃, s, 3H).

### 5. Synthesis of 4-chloro-5-methyl-[1,10]phenanthroline-2-carboxylic acid methyl ester (58)

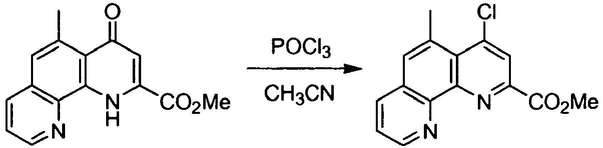

POCl₃ (3.7 mL, 40.4 mmol) was added into a solution of 5-methyl-4-oxo-1,4-dihydro-[1,10]phenanthroline-2-carboxylic acid methyl ester **57** (5.7 g, 21.2 mmol) in CH₃CN (80 mL). The reaction mixture was stirred at room temperature for 5 hours, diluted with CH₂Cl₂ and solid K₂CO₃ was added until pH 10. Water and CH₂Cl₂ were added and the mixture was transferred to a separatory funnel. The organic layer was washed with brine, dried (Na₂SO₄), filtered and evaporated. The residue was purified by flash chromatography (SiO₂, MeOH/CH₂Cl₂, 1-20%) to yield the chloro derivative **58** (4.1 g, 68%) as a light brown solid.

¹H-NMR (400 MHz, CDCl₃, ppm): 9.15 (H-9, dd, 1H, J=1.5 and 4.3 Hz), 8.43 (H-3, s, 1H), 8.10 (H-7, dd, 1H, J=1.5 and 8.1 Hz), 7.66 (H-6, s, 1H), 7.61 (H-8, dd, 1H, J=4.3 and 8.1 Hz), 4.07 (OCH₃, s, 3H), 3.06 (CH₃, s, 3H).

### 6. Synthesis of (4-chloro-5-methyl-[1,10]phenanthrolin-2-yl)-methanol (59)

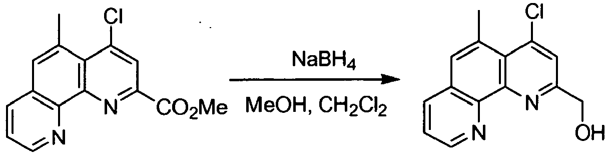

Solid sodium borohydride (0.24 g, 6.3 mmol) was added portionwise into a solution of 4-chloro-5-methyl-[1,10]phenanthroline-2-carboxylic acid methyl ester **58** (1.00 g, 3.5 mmol) in a mixture of MeOH/CH₂Cl₂ (1:1, 20 mL) previously cooled at 0°C in an ice bath. The addition was kept with stirring at 0°C for 3 hours. The reaction mixture was allowed to warm to room temperature and water was added. The mixture was extracted with CH₂Cl₂ and the organic layer was separated, washed with brine, dried (Na₂SO₄), filtered and evaporated till dryness. The residue was treated with ethyl ether affording alcohol **59** as a dark red solid (0.77 g, 85%).

¹H-NMR (400 MHz, CDCl₃, ppm): 9.04 (H-9, d, 1H, J= 4.2 Hz), 8.12 (H-7, dd, 1H, J=1.3 and 8.0 Hz), 7.78 (H-3, s, 1H), 7.58 (H-8, dd, 1H, J=4.2 and 8.0 Hz), 7.56 (H-6, s, 1H), 5.13 (OCH₂, s, 2H), 3.07 (OCH₃, s, 3H).

### 7. Synthesis of (5-methyl-4-methylsulfanyl-[1,10]phenanthrolin-2-yl)-methanol (60)

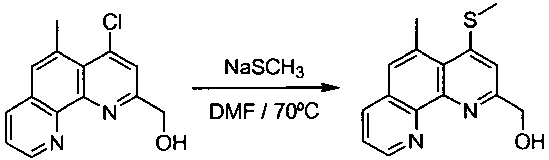

To a solution of (4-chloro-5-methyl-[1,10]phenanthrolin-2-yl)-methanol **59** (100.0 mg, 0.4 mmol) in anhydrous DMF (2 mL) at 70°C a solution of sodium thiomethoxide (82.0 mg, 1.2 mmol) in anhydrous DMF (3 mL) was added. Stirring was kept at 70°C for 15 minutes and water was added. The mixture was diluted in MeOH/CH₂Cl₂ (1:1 and the organic layer was separated, washed with brine, dried (Na₂SO₄), filtered and evaporated. The residue was purified by flash chromatography (SiO₂, 7N NH₃OH in MeOH/CH₂Cl₂, 0-50%) yielding the desired product **60** as a light brown solid (30.3 mg, 29%).

¹H-NMR (400 MHz, MeOD, ppm): 8.97 (H-9, dd, 1H, J= 1.6 and 4.4 Hz), 8.25 (H-7, dd, 1H, J=1.7 and 8.1 Hz), 7.68 (H-8, dd, 1H, J=4.4 and 8.1 Hz), 7.55 (H-3, s, 1H), 7.52 (H-6, s, 1H), 4.95 (OCH₂, s, 2H), 3.05 (SCH₃, s, 3H), 2.64 (CH₃, s, 3H).

### 8. Synthesis of 5-methyl-4-methylsulfanyl-[1,10]phenanthroline-2-carbaldehyde (61)

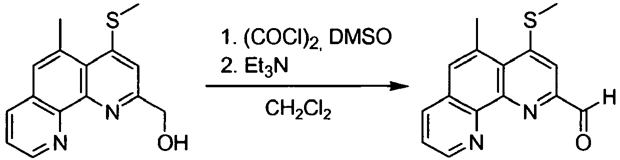

To a 2.0 M solution of oxalyl chloride in CH₂Cl₂ (600 µl, 1.2 mmol) at -78°C, anhydrous DMSO (170 µl, 2.4 mmol) was added and the mixture was stirred at -78°C for 20 minutes. Then, a solution of (5-methyl-4-methylsulfanyl-[1,10]phenanthrolin-2-yl)-methanol **60** (161.0 mg, 0.6 mmol) in anhydrous CH₂Cl₂ (3 mL) was added and stirring was kept at -78°C for 50 minutes. Finally, triethylamine (0.5 ml, 3.6 mmol) was added at -78°C, and the reaction mixture was allowed to warm to room temperature. The mixture was diluted with CH₂Cl₂ and washed with water. The organic layer was separated, dried (Na₂SO₄), filtered and evaporated. The residue was purified by flash chromatography (SiO₂, MeOH/CH₂Cl₂, 0-50%), yielding aldehyde **61** as a light brown solid (51.4 mg, 32%).

¹H-NMR (400 MHz, CDCl₃, ppm): 10.48 (CHO, s, 1H), 9.20 (H-9, dd, 1H, J= 1.7 and 4.3 Hz), 8.15 (H-7, dd, 1H, J=1.7 and 8.1 Hz), 7.98 (H-3, s, 1H), 7.66 (H-6, s, 1H), 7.65 (H-8, dd, 1H, J=4.3 and 8.1 Hz), 3.16 (SCH₃, s, 3H), 2.69 (CH₃, s, 3H).

### 9. Synthesis of 5-methyl-4-methylsulfanyl-[1,10]phenanthroline-2-carbaldehyde oxime (D)

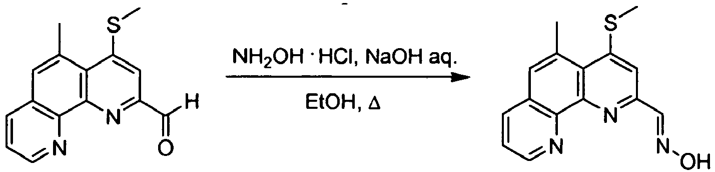

A solution of hydroxylamine hydrochloride (133.0 mg, 1.9 mmol) in water (2 ml) was added into a solution of 5-methyl-4-methylsulfanyl-[1,10]phenanthroline-2-carbaldehyde **61** (51.4 mg, 0.2 mmol) in a mixture of ethanol/ CH₂Cl₂ (5/1.5, 6.5 mL). The stirring was kept at room temperature for 16 hours. The reaction mixture was diluted with water and CH₂Cl₂, and the organic layer was separated, dried (Na₂SO₄), filtered and evaporated. The residue was purified by flash chromatography (SiO₂, MeOH/CH₂Cl₂, 0-5,0%), yielding the aldoxime **D** as a light brown solid (8.4 mg, 15%).

¹H-NMR (400 MHz, CDCl₃/MeOD, 3/1, ppm): 9.00 (H-9, dd, 1H, J= 1.5 and 4.0 Hz), 8.50 (H-3, s, 1H), 8.28 (H-7, dd, 1H, J= 1.4 and 8.0 Hz), 7.96 (H-6, s, 1H), 7.70 (H-8, dd, 1H, J= 4.0 and 8.0 Hz), 7.62 (CH=, s, 1H), 3.15 and 3.13 (SCH₃, s and s, 3H), 2.72 and 2.66 (CH₃, s and s, 3H).

### Example 6

### Synthesis of Phenanthroline Derivative A

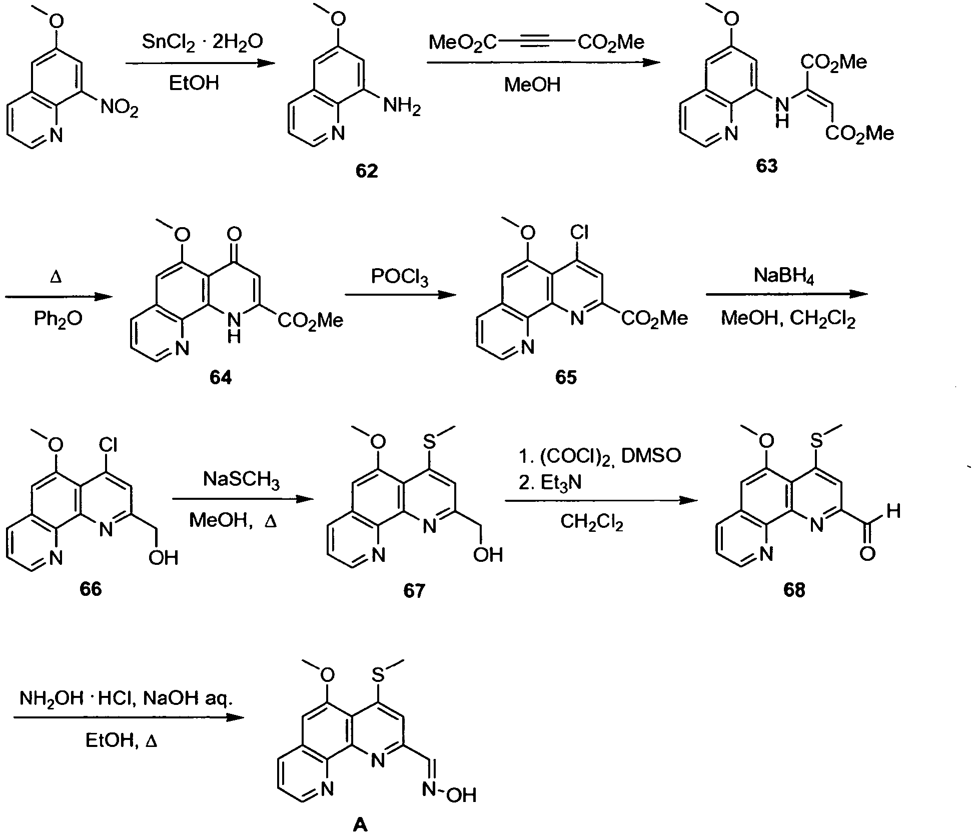

### 1. Synthesis of 6-methoxy-quinolin-8-ylamine (62)

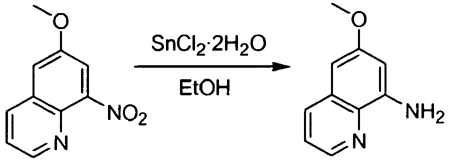

SnCl₂·2H₂O (39.78 g, 176.3 mmol) was added to a solution of 6-methoxy-8-nitro-quinoline (18.00 g, 88.1 mmol) in ethanol (200 mL). The mixture was heated to reflux for approximately two hours and the resulting solution was basified with 1N NaOH. The tin-salts were filtered off and the mother liquors were extracted several times with CH₂Cl₂. The combined organic layers were dried (Na₂SO₄) and evaporated to give 6-methoxy-8-amino-quinoline **62** (15.00 g, 98%) as a brown solid.

¹H NMR (400 MHz, CDCl₃, ppm): 8.59 (dd, *J* = 4.21, 1.65 Hz, 1H), 7.94 (dd, *J* = 8.29, 1.64 Hz, 1H), 7.31 (dd, *J* = 8.28, 4.20 Hz, 1H), 6.58 (d, *J* = 2.57 Hz, 1H), 6.47 (d, *J* = 2.57 Hz, 1H), 3.87 (s, 3H).

¹³C NMR (100 MHz, CDCl₃, ppm): 158.79, 145.01, 144.97, 135.38, 134.72, 129.83, 121.77, 101.55, 94.53, 55.20.

### 2. Synthesis of 2-(6-methoxy-quinolin-8-ylamino)-but-2-enedioic acid dimethyl ester (63)

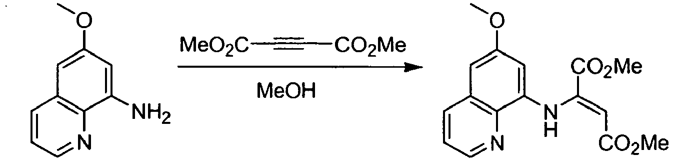

Dimethylacetylenedicarboxylate (6.0 mL, 48.9 mmol) was added to a solution of 6-methoxy-quinolin-8-ylamine (7.75 g, 44.5 mmol) in MeOH (40 mL). The mixture was stirred at room temperature for 3 hours. After solvent removal, the residue was purified by flash chromatography (SiO₂, ethyl acetate/hexane, 3:1) to obtain pure compound **63** (4.20 g, 30%) as a yellow solid.

¹H NMR (400 MHz, CDCl₃, ppm): 10.95 (s, 1H), 8.73 (dd, J = 4.22, 1.65 Hz, 1H), 7.98 (dd, J = 8.30, 1.63 Hz, 1H), 7.36 (dd, J = 8.28, 4.22 Hz, 1H), 6.70 (d, J = 2.48 Hz, 1H), 6.56 (d, J = 2.46 Hz, 1H), 5.55 (s, 1H), 3.86 (s, 3H), 3.78 (s, 6H).

¹³C NMR (100 MHz, CDCl₃, ppm): 169.07, 165.01, 157.69, 146.42, 145.83, 137.75, 136.34, 134.71, 129.50, 122.08, 106.64, 99.07, 96.36, 55.41, 52.85, 51.36.

### 3. Synthesis of 5-methoxy-4-oxo-1,4-dihydro-[1,10]phenanthroline-2-carboxylic acid methyl ester (64)

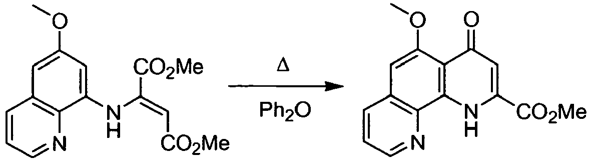

Quinoline derivative **63** (4.20 g, 13.28 mmol) was heated in diphenylether (40 mL) at 240°C for 20 minutes. The solution was cooled at room temperature, hexane was added to obtain a brown paste which was filtered and then eluted with hot methanol. The methanolic solution was concentrated to obtain a dark brown solid, that was triturated with ethyl ether to afford the desired product **64** (0.75 g, 20%) as a light brown solid

¹H NMR (400 MHz, CDCl₃, ppm) 8.78 (d, J = 4.13 Hz, 1H), 8.08 (dd, J = 8.23, 1.31 Hz, 1H), 7.57 (dd, J = 8.23, 4.30 Hz, 1H), 7.18 (s, 1H), 6.82 (s, 1H), 4.07 (s, 3H), 4.06 (s, 3H), 2.17 (s, 1H)

¹³C NMR (100 MHz, CDCl₃, ppm) 146.77, 146.69, 141.67, 134.59, 134.53, 130.16, 124.68, 118.86, 117.30, 98.81, 86.29, 53.62.

### 4. Synthesis of 4-chloro-5-methoxy-[1,10]phenanthroline-2-carboxylic acid methyl ester (65)

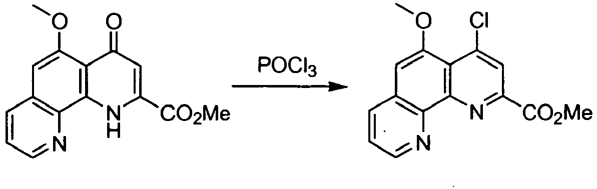

POCl₃ (15 mL) was added to the phenanthroline derivative **65** (0.89 g, 3.13 mmol) and the mixture was stirred at room temperature for 3 hours. After evaporation at reduced pressure, the residue was dissolved in CH₂Cl₂ and sequentially washed with saturated NaHCO₃ and brine, dried (Na₂SO₄) and evaporated to afford pure chlorophenanthroline intermediate **65** (1.00 g 100%) as a brown solid.

¹H NMR (400 MHz, CDCl₃, ppm): 9.28 (d, J = 3.63 Hz, 1H), 8.51 (d, J = 7.98 Hz, 1H), 8.46 (s, 1H), 7.84 (dd, J = 7.02, 4.58 Hz, 1H), 7.29 (s, 1H), 4.13 (s, 3H), 4.12 (s, 3H)

¹³C NMR (100 MHz, CDCl₃, ppm): 164.48, 154.82, 147.69, 143.49, 139.01, 130.02, 129.68, 127.43, 124.88, 123.21, 118.84, 103.96, 56.38, 53.58

### 5. Synthesis of (4-chloro-5-methoxy-[1,10]phenanthrolin-2-yl)-methanol (66)

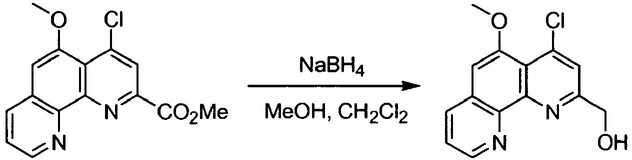

Solid sodium borohydride (0.25 g, 6.6 mmol) was added portion wise to a solution of 4-chloro-5-methoxy-[1,10]phenanthroline-2-carboxylic acid methyl ester **65** (1.00 g, 3.3 mmol) in a mixture of MeOH/CH₂Cl₂ (1:5, 50 mL) cooled at 0°C. The mixture was stirred at 0°C for 1 hour and at room temperature for 18 hours. The solvent was removed and the residue was dissolved in CH₂Cl₂ and washed sequentially with water and brine, dried (Na₂SO₄) and evaporated to afford pure alcohol **66** (710 mg 78%) as a brown solid.

¹H NMR (400 MHz, CDCl₃, ppm): 8.91 (s, 1H), 8.09 (dd, J = 8.06, 1.19 Hz, 1H), 7.74 (s, 1H), 7.54 (dd, J = 8.03, 4.40 Hz, 1H), 6.97 (s, 1H), 5.10 (s, 2H), 4.06 (s, 3H).

¹³C NMR (100 MHz, CDCl₃, ppm): 160.86, 153.98, 147.66, 141.89, 134.82, 129.69, 129.29, 123.87, 123.53, 120.14, 118.85, 102.34, 65.05, 55.74.

### 6. Synthesis of (5-methoxy-4-methylsulfanyl-[1,10]phenanthrolin-2-yl)-methanol (67)

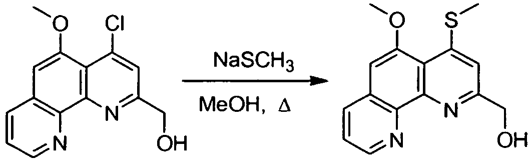

To a solution of the chloro derivative **66** (710 mg, 2.6 mmol) in anhydrous MeOH (10 mL), solid sodium thiometoxide (906 mg, 12.9 mmol) was added. The resulting mixture was heated to reflux for 8 hours. The solvent was removed at reduced pressure, the residue was dissolved in CH₂Cl₂ and washed with saturated NaHCO₃ and brine, dried (Na₂SO₄), filtered and concentrated to obtain pure compound **67** (500 mg, 70%) as a brown solid.

¹H NMR (400 MHz, CDCl₃, ppm): 8.87 (d, J = 2.86 Hz, 1H), 8.05 (d, J = 1.17 Hz, 1H), 7.47 (dd, J = 7.95, 4.28 Hz, 1H), 7.39 (s, 1H), 6.88 (s, 1H), 5.13 (s, 2H), 4.07 (s, 3H), 2.49 (s, 3H)

¹³C NMR (100 MHz, CDCl₃, ppm): 159.49, 155.12, 150.90, 147.51, 134.36, 129.69, 128.98, 123.35, 123.17, 118.85, 114.45, 100.82, 65.59, 55.37, 16.168.

### 7. Synthesis of 5-methoxy-4-methylsulfanyl-[1,10]phenanthroline-2-carbaldehyde (68)

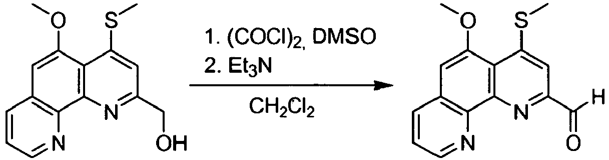

To a solution of 2.0 M of oxalyl chloride in CH₂Cl₂ anhydrous (1.4 mL, 2.8 mmol) at -78°C, a solution of anhydrous DMSO (0.4 mL, 5.6 mmol) in anhydrous CH₂Cl₂ (2 mL) was added dropwise under nitrogen. After 20 minutes of stirring at -78°C, a solution of the alcohol derivative **67** (400 mg, 1.4 mmol) in dry CH₂Cl₂ (5 mL) was added dropwise. The solution was stirred for 50 minutes at -78°C and triethylamine (1.2 mL, 8.4 mmol) was added slowly. After 5 minutes the mixture was allowed to warm to room temperature and stirred for 5 hours. Water was added and the mixture was tranferred to a separatory funnel, the organic layer was then washed with water and brine, dried (Na₂SO₄) and concentrated to afford pure aldehyde **68** (375 mg, 94%) as a brown solid.

¹H NMR (400 MHz, CDCl₃, ppm): 10.49 (s, 1H), 9.11 (dd, J = 4.35, 1.68 Hz, 1H), 8.15 (dd, J = 8.10, 1.67 Hz, 1H), 7.99 (s, 1H), 7.63 (dd, J = 8.09, 4.36 Hz, 1H), 7.09 (s, 1H), 4.12 (s, 3H), 2.61 (s, 3H)

¹³C NMR (100 MHz, CDCl₃, ppm): 194.355, 152.809, 150.630, 148.493, 134.554, 134.510, 129.708, 129.133, 123.955, 123.195, 118.867, 113.629, 103.696, 55.622, 16.366.

### 8. Synthesis of 5-methoxy-4-methylsulfanyl-[1,10]phenanthroline-2-carbaldehyde oxime (A)

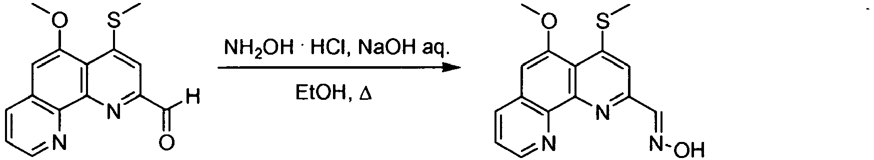

To a suspension of aldehyde **68** (330 mg, 1.16 mmol) in ethanol (16 mL), a solution of hydroxylamine hydrochloride (801 mg, 11.6 mmol) in water (8 mL) was added and the mixture was heated at 60°C. A solution of 10% NaOH was added until pH 6 and the reaction was stirred at 60°C for 1 hour and cooled at 0°C. A precipitate formed which was filtered and washed with water to obtain the desired aldoxime **A** (233 mg, 70%) as a light brown solid.

¹H NMR (400 MHz, CDCl₃, ppm): 11.98 (s, 1H), 8.91 (s, 1H), 8.31 (d, J = 6.68 Hz, 2H), 7.86 (s, 1H), 7.68 (dd, J = 7.53, 3.71 Hz, 1H), 7.35 (s, 1H), 4.05 (s, 3H), 2.54 (s, 3H)

¹³C NMR (100 MHz, CDCl₃. ppm): 154.053, 150.819, 14.9.909, 148.876, 147.384, 134.456, 129.938, 128.818, 123.710, 120.327, 118.498, 112.746, 102.459, 55.695, 15.324.

MS (ES-): m/z = 298.

### BIOLOGY

### Example 7

### Toxicity

The potential effects on cell viability of the tested compounds were assessed in SH-SY5Y human neuroblastoma cells, by quantification of Lactate dehydrogenase (LDH) activity release. SH-SY5Y human neuroblastoma cells were seeded into 96-well culture plates at 10⁴ cells/well. The medium was then removed and the cells incubated with different concentrations of the compounds during 24 h. The compounds were tested at increasing concentrations starting from 1µM up to a maximum of 1.000µM, in fresh culture medium, in order to find the minimum concentration at which the compounds significantly compromise cell viability. After 24 h, the medium was removed and cells attached to the bottom of the well were lysed by adding 50 µl of Krebs-Hepes; Triton X-100 1% during 5 minutes at room temperature. For LDH release quantification, the Roche cytotoxicity detection kit (Cat. No. 11 644 793 001) was used. The LDH activity was measured by its absorbance at 492 nm with reference wavelength 620 nm.

In **Table 2,** for each compound the maximum concentration at which cell viability was tested is indicated in the second column. In the third column, it is indicated whether at this maximum concentration the compound affected or not cell viability (>20% of cell death). None of the compounds showed any effect on cell viability at the concentration for which activity was found, in most of the cases even at a 1000-fold concentration. Thus, these results clearly indicate that the tested compounds do not affect cell viability at concentrations well above the active ones.

**Table 2**

| **Compound** | **Maximum concentration tested for cell viability** | **Yes/No** |
|---|---|---|
| A | 100 µM | Y |
| B | 1000 µM | N |
| C | 1000 µM | N |
| D | 1000 µM | N |
| E | 100 µM | Y |
| F | 1000 µM | N |

### Example 8

### Protection against hydrogen peroxide-induced cell death

The aim of this assay is to determine the neuroprotective effect of the compounds of formula (I), when human neuroblastoma cells are exposed to oxidative stress induced by hydrogen peroxide, which is highly deleterious to the cell and its accumulation causes oxidation of cellular targets such as DNA, proteins, and lipids leading to mutagenesis and cell death.

SH-SY5Y human neuroblastoma cells are seeded into 96- well culture plate at a density of 10⁴ cells/well. Cells are exposed to different concentrations of the compound one hour before the treatment with H₂O₂ 100 µM during 24 h. 5 mM N-acetylcysteine (NAC), a known anti-oxidant agent was used as a positive control, and preincubated 1 hour before the treatment with H₂O₂. After 24 h, the medium is removed and cells attached to the bottom of the well are lysed by adding 50 µl of Triton X-100 1 % in Krebs-Hepes during 5 minutes at room temperature. For LDH release quantification, Roche cytotoxicity detection kit (Cat. No. 11 644 793 001) was used.

The minimum concentration of Compounds **A-E** for which protection against H₂O₂ was determined are shown in **Table 3.**

**Table 3**

| **Compound** | **Protect H₂O₂** |
|---|---|
| A | 0,05 µM |
| B | 0,05 µM |
| C | 0,005 µM |
| D | 0,5 µM |
| E | 0,05 µM |

### Example 9

### Protection against 6-OHDA- induced cell death

The aim of this experiment is to determine the protective effect of the compounds of formula (I) against the toxicity caused by 6-OHDA. This toxin induces a cell death similar to which occurs in Parkinson's disease, destroying dopaminergic neurons *("MPTP and 6-hydroxydopamine-induced neurodegeneration* as *models for Parkinson's disease: neuroprotective strategies";* Grunblatt E, et al.; J Neurol. 2000 Apr; 247 Suppl 2:II95-102).

Two or three days before the experiment, the SH-SY5Y human neuroblastoma cells are seeded into 96- well culture plate at a density of 10⁴ cells/well. Cells are exposed to the treatment with 6-OHDA and, finally, cell death is measured by LDH quantification. As positive control we used NAC.

NAC and the compound of formula (I) are preincubated during 2 hours before the treatment with 6-OHDA 75 µM during 16 hours. The assay is performed in medium containing 10% Foetal bovine serum.

The neuroprotective results against cellular death induced by 6-OHDA are shown in **Table 4.** For each compound the minimum concentration of compound of formula (I) at which a neuroprotective effect is shown.

**Table 4**

| **Compound** | **Protection 6-OHDA** |
|---|---|
| A | 0,05 µM |
| B | 0,05 µM |
| C | 0,5 µM |
| D | 0,05 µM |
| E | 0,5 µM |
| F | 10 µM |

### Example 10

### Neuroprotection against Aβ toxicity

In order to evaluate potential neuroprotection of compounds, SH-SY5Y cells, cultured in 96-well plates, were pre-treated for 1 hour with the compound at different concentrations and then exposed 24 hours to 200 µM Aβ₂₅₋₃₅ (Neosystem) to induce extensive oxidative stress and cell death. The ability of the compound of protecting against this toxicity is then evaluated by measuring intracellular LDH, using the colorimetric LDH assay.

It is widely accepted that the neurotoxic activity of Aβ resides within amino acids 25-35 (see e.g. Yankner BA et al., (1990) Neurotrophic and neurotoxic effects of amyloid β protein: reversal by tachykinin neuropeptides; Science 250:279-282).

In **Table 5,** the minimum concentration at which the tested compounds showed neuroprotection against Aβ₂₅₋₃₅ toxicity is shown.

**Table 5**

| **Compound** | **Protection β-Amiloyd** |
|---|---|
| **A** | 5 µM |
| **B** | 5 µM |
| **C** | 5 µM |
| **D** | 0,5 µM |
| **E** | 10 µM |

### Example 13

### Inhibition of Aβ(1-40) secretion

To quantitate Aβ secretion ELISA-based method was used. The assay consists in detection of antigen by selective monoclonal anti-Aβ-antibodies at two different epitopes forming a "Sandwich-complex", that is detected by colorimetric measure due to the binding of a secondary antibody conjugated with peroxidase that catalyses the conversion of a substrate or chromogen, TMB, into a coloured product, directly proportional to the peptide quantity in the sample. The Aβ production has been analyzed by ELISA, using a colorimetric commercial kit: H-amuloid b-30 ELISA (The Genetics Company).

Aβ (1-40) were quantified from cellular supernatants. An APP-transfected cell line has been employed for the experiments: CHO7W (stably transfected with human APP₇₅₁ wt cDNA). The cells were grown in a culture medium consisting of DMEM supplemented with 2% Fetal bovine serum, 1% penicillin-streptomycin, 1% L-glutamine and 200 µg/ml G418. Cells are seeded in 96-well culture microplate, at 5000 cells/well and treatment with different compounds at different concentrations is performed 24 hour after seeding.

In **Table 6** the minimum concentration for each tested compound at which the compound inhibits beta-amyloid secretion is shown.

**Table 6**

| **Compound** | **inhibition Aβ secretion** |
|---|---|
| **A** | 0,1 µM |
| **B** | 1 µM |
| **C** | 0,1 µM |
| **D** | 1 µM |
| **E** | 10 µM |

## Claims

1. A compound of formula (I): 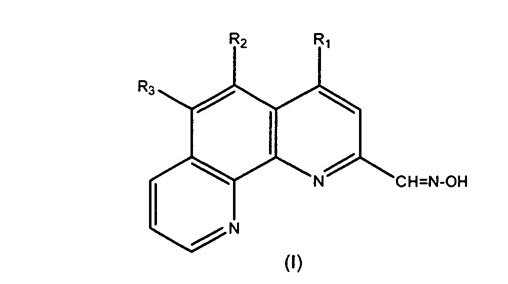 wherein
R¹ is selected from -O-R⁴ and -S-R⁵, wherein R⁴ and R⁵ are selected from H and C₁-C₆ alkyl,
R² is selected from hydrogen, halogen, C₁-C₆-alkoxyl, C₁-C₆ alkyl and -O-(CH₂)ₙ-O-R⁶ wherein n is selected from 1, 2, 3, 4, 5, 6, and R⁶ is C₁-C₆ alkyl,
R³ is selected from hydrogen and C₁-C₆ alkoxyl,
with the proviso that one of R² and R³ is H and the other is different from H,
or any salt or solvate or stereoisomer or tautomer thereof.

2. A compound according to claim 1, wherein R⁴ and R⁵ are selected from H and methyl.

3. A compound according to any one of claims 1 and 2, wherein R² is selected from hydrogen, fluor, methyl, methoxy and -O-(CH₂)₂-O-CH₃.

4. A compound according to any one of claims 1 to 3, wherein R³ is selected from hydrogen and methoxyl.

5. A compound according to any one of claims 1 to 4, wherein the double bond of the oxime group -CH=NOH presents E conformation.

6. A compound according to any one of claims 1 to 5, wherein the compound of formula (I) is selected from the following compounds:
5-Methoxy-4-methylsulfanyl-[1,10]phenanthroline-2-carbaldehyde oxime 5-Fluoro-4-methoxy-[1,10]phenanthroline-2-carbaldehyde oxime
4-Methoxy-5-(2-methoxy-ethoxy)-[1,10]phenanthroline-2-carbaldehyde oxime
5-Methyl-4-methylsulfanyl-[1,10]phenanthroline-2-carbaldehyde oxime
6-Methoxy-4-methylsulfanyl-[1,10]phenanthroline-2-carbaldehyde oxime
4-Hydroxy-6-methoxy-[1,10]phenanthroline-2-carbaldehyde oxime

7. A compound as defined in any one of claims 1 to 6 for use as a medicament.

8. A compound of formula (I) as defined in any one of claims 1 to 6 for use in the treatment or prophylaxis of a neurodegenerative or haematological disease or condition.

9. A compound of formula (I) as defined in any one of claims 1 to 6 for use in the treatment or prevention of cancer

10. A compound for use in the treatment or prophylaxis of a neurodegenerative disease according to claim 8, wherein the neurodegenerative disease is selected from Alzheimer's Disease, Parkinson's Disease, amyotrophic lateral sclerosis (ALS), schizophrenia, Huntington's Disease, brain injuries, such as stroke and ischemia, multiple sclerosis, epilepsy, Friedreich's Ataxia, spongiform encephalopaties, amyloidosis, vascular dementia, tauophaties, progressive supranuclear palsy, corticobasal degeneration, frontotemporal lobular degeneration, subacute sclerosing panencephalitic parkinsonism, postencephalitic parkinsonism, pugilistic encephalitis, guam parkinsonism-dementia complex, Pick's disease, frontotemporal dementia, AIDS associated dementia, multiple sclerosis, mood disorders such as depression, schizophrenia and bipolar disorders, promotion of functional recovery post stroke and brain injury, especially traumatic brain injury.

11. A compound for use in the treatment or prephylaxis of haematological disease according to claim 8, wherein the haematological disease is selected from thalassaemia, anaemia, aplastic anaemia, Diamond-Blackfan anemia, sickle cell disease, hematologic disorders which require regular red cell transfusions, myelodysplastic syndrome, iron-induced cardiac dysfunction, iron-induced heart failure, and diabetes.

12. A compound for use in the treatment or prevention of cancer according to claim 9, wherein the cancer is selected from intestines, liver, gastric, breast, lung, ovary, prostate, brain glioma, lymph, skin, pigment, thyroid gland, leukemia and multiple bone marrow cancer.

13. A pharmaceutical composition comprising at least one compound of formula (I) as defined in any one of claims 1 to 6, its salts or solvates or tautomers thereof, and at least one pharmaceutically acceptable carrier.

14. Use of a compound of formula (I) as defined in any one of claims 1 to 6, or any salt or solvate thereof, as reagent for biological assays, preferably as a reactive for pharmacokinetic assays, blood brain barrier crossing assays, chelation assays, for essays on protection against hydrogen peroxide-induced cell death, protection against 6-OHDA-induced cell death, neuroprotection against Aβ toxicity and inhibiton of beta-amyloid secretion.

## Patentansprüche

1. Verbindung der Formel (I): 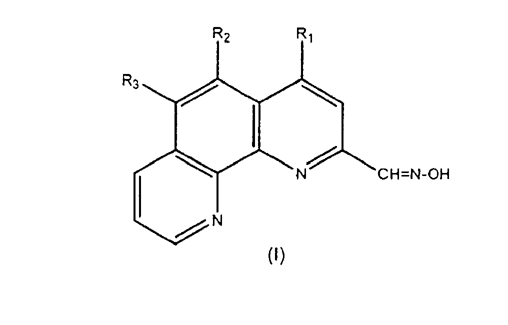 wobei
R¹ aus -O-R⁴ und -S-R⁵ ausgewählt ist, wobei R⁴ und R⁵ aus H und C₁-C₆-Alkyl ausgewählt sind;
R² aus Wasserstoff, Halogen, C₁-C₆-Alkoxy, C₁-C₆-Alkyl und -O-(CH₂)ₙ-O-R⁶ ausgewählt ist, wobei n aus 1, 2, 3, 4, 5, 6 ausgewählt ist und R⁶ = C₁-C₆-Alkyl ist;
R³ aus Wasserstoff und C₁-C₆-Alkoxy ausgewählt ist;
mit der Maßgabe, dass eines von R² und R³ = H ist und das andere von H verschieden ist;
oder ein Salz oder Solvat oder Stereoisomer oder Tautomer davon.

2. Verbindung gemäß Anspruch 1, wobei R⁴ und R⁵ aus H und Methyl ausgewählt sind.

3. Verbindung gemäß einem der Ansprüche 1 und 2, wobei R² aus Wasserstoff, Fluor, Methyl, Methoxy und -O-(CH₂)₂-O-CH₃ ausgewählt ist.

4. Verbindung gemäß einem der Ansprüche 1 bis 3, wobei R³ aus Wasserstoff und Methoxy ausgewählt ist.

5. Verbindung gemäß einem der Ansprüche 1 bis 4, wobei die Doppelbindung der Oximgruppe -CH=NOH eine E-Konformation aufweist.

6. Verbindung gemäß einem der Ansprüche 1 bis 5, wobei die Verbindung der Formel (I) aus den folgenden Verbindungen ausgewählt ist:
5-Methoxy-4-methylsulfanyl[1,10]phenanthrolin-2-carbaldehydoxim
5-Fluor-4-methoxy[1,10]phenanthrolin-2-carbaldehydoxim
4-Methoxy-5-(2-methoxyethoxy)[1,10]phenanthrolin-2-carbaldehydoxim
5-Methyl-4-methylsulfanyl[1,10]phenanthrolin-2-carbaldehydoxim
6-Methoxy-4-methylsulfanyl[1,10]phenanthrolin-2-carbaldehydoxim
4-Hydroxy-6-methoxy[1,10]phenanthrolin-2-carbaldehydoxim.

7. Verbindung gemäß einem der Ansprüche 1 bis 6 zur Verwendung als Medikament.

8. Verbindung der Formel (I) gemäß einem der Ansprüche 1 bis 6 zur Verwendung bei der Behandlung oder Prophylaxe einer neurodegenerativen oder hämatologischen Krankheit oder eines neurodegenerativen oder hämatologischen Zustands.

9. Verbindung der Formel (I) gemäß einem der Ansprüche 1 bis 6 zur Verwendung bei der Behandlung oder Prävention von Krebs.

10. Verbindung zur Verwendung bei der Behandlung oder Prophylaxe einer neurodegenerativen Krankheit gemäß Anspruch 8, wobei die neurodegenerative Krankheit aus Alzheimer-Krankheit, Parkinson-Krankheit, amyotropher Lateralsklerose (ALS), Schizophrenie, Huntington-Krankheit, Hirnverletzungen, wie Schlaganfall und Ischämie, multipler Sklerose, Epilepsie, Friedreich-Ataxie, spongiformen Enzephalopathien, Amyloidose, vaskulärer Demenz, Tauopathien, progressiver supranukleärer Blickparese, kortikobasaler Degeneration, Frontotemporallappen-Degeneration, subakuter sklerosierender Panenzephalitis, postenzephalitischem Parkinson-Syndrom, Boxer-Enzephalopathie, ALS-Parkinson-Demenz-Komplex, Pick-Krankheit, frontotemporaler Demenz, AIDS-Demenz, multipler Sklerose, Gemütsstörungen, wie Depression, Schizophrenie und bipolaren Störungen, Förderung der funktionellen Erholung nach Schlaganfall und Gehirnverletzung, insbesondere traumatischer Gehirnverletzung, ausgewählt ist.

11. Verbindung zur Verwendung bei der Behandlung oder Prophylaxe einer hämatologischen Krankheit gemäß Anspruch 8, wobei die hämatologische Krankheit aus Thalassämie, Anämie, aplastischer Anämie, Diamond-Blackfan-Anämie, Sichelzellanämie, hämatologischen Störungen, die regelmäßige Erythrocytentransfusionen erfordern, myelodysplastischem Syndrom, eiseninduzierter kardialer Dysfunktion, eiseninduziertem Herzversagen und Diabetes ausgewählt ist.

12. Verbindung zur Verwendung bei der Behandlung oder Prävention von Krebs gemäß Anspruch 9, wobei die Krebserkrankung aus Darm-, Leber-, Magen-, Brust-, Lungen-, Eierstock-, Prostatakrebs, Hirngliom, Lymph-, Haut-, Pigment-, Schilddrüsenkrebs, Leukämie und multiplem Knochenmarkkrebs ausgewählt ist.

13. Pharmazeutische Zusammensetzung, die wenigstens eine Verbindung der Formel (I), wie sie in einem der Ansprüche 1 bis 6 definiert ist, deren Salze oder Solvate oder Tautomere sowie wenigstens einen pharmazeutisch annehmbaren Träger umfasst.

14. Verwendung einer Verbindung der Formel (I), wie sie in einem der Ansprüche 1 bis 6 definiert ist, oder eines Salzes oder Solvats davon als Reagens für biologische Assays, vorzugsweise als Reagens für pharmakokinetische Assays, Blut-Hirn-Schranke-Passage-Assays, Chelatisierungsassays, für Assays auf Schutz vor Wasserstoffperoxidinduziertem Zelltod, Schutz vor 6-OHDA-induziertem Zelltod, Neuroprotektion gegen Aß-Toxizität und Hemmung der beta-Amyloid-Sekretion.

## Revendications

1. Composé de formule (I) : 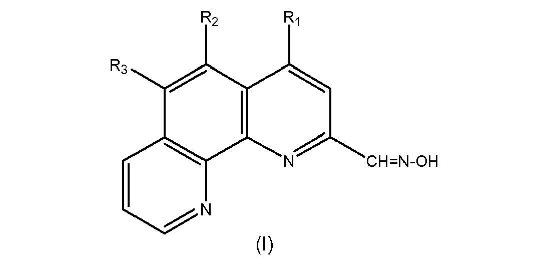 **caractérisé en ce que** :
- R¹ est choisi entre -O-R⁴ et -S-R⁵, où R⁴ et R⁵ sont choisis entre de l'hydrogène et un alkyl C₁-C₆,
- R² est choisi parmi l'hydrogène, un halogène, un alkoxyle C₁-C₆, un alkyle C₁-C₆ et -O-(CH2)ₙ-O-R⁶, où n est choisi entre 1, 2, 3, 4, 5, 6 et R⁶ est un alkyl C₁-C₆,
- R³ est choisi entre l'hydrogène et un alkoxyle C₁-C₆,
avec la condition qu'un des groupes parmi R² et R³ est de l'hydrogène et l'autre n'est pas de l'hydrogène,
ou ni aucun sel ou solvaté ou stéréoisomère ou tautomère de celui-ci.

2. Composé selon la revendication 1, **caractérisé en ce que** R⁴ et R⁵ sont choisis entre l'hydrogène et le groupe méthyle.

3. Composé selon l'une quelconque des revendication 1 et 2, **caractérisé en ce que** R² est choisi parmi l'hydrogène, le fluor, un groupe méthyle, méthoxyle et -O-(CH₂)₂-O-CH₃.

4. Composé selon l'une quelconque des revendications 1 à 3, **caractérisé en ce que** R³ est choisi entre l'hydrogène et le groupe méthoxyle.

5. Composé selon l'une quelconque des revendications 1 à 4, **caractérisé en ce que** la double liaison du groupe oxime -CH=NOH présente une conformation en E.

6. Composé selon l'une quelconque des revendications 1 à 5, **caractérisé en ce que** le composé de formule (I) est choisi parmi les composés suivantes :
- oxime de 5-méthoxy-4-méthylsulfanyl-{1,10}phénanthroline-2-carbaldéhyde,
- oxime de 5-fluoro-4-méthoxy-{1,10}phénanthroline-2-carbaldéhyde,
- oxime de 4-méthoxy-5-(2-méthoxy-éthoxy)-{1,10}phénanthroline-2-carbaldéhyde,
- oxime de 5-méthyl-4-méthylsulfanyl-{1,10}phénanthroline-2-carbaldéhyde,
- oxime de 6-méthoxy-4-méthylsulfanyl-{1,10}phénanthroline-2-carbaldéhyde,
- oxime de 4-hydroxy-6-méthoxy-{1,10}phénanthroline-2-carbaldéhyde.

7. Composé selon l'une quelconque des revendications 1 à 6 destiné à être utilisé comme médicament.

8. Composé de formule (I) selon l'une quelconque des revendications 1 à 6 destiné à être utilisé dans le traitement ou la prophylaxie d'une maladie ou d'un état neuro-dégénératif ou hématologique.

9. Composé de formule (I) selon l'une quelconque des revendications 1 à 6 destiné à être utilisé dans le traitement ou la prévention du cancer.

10. Composé destiné à être utilisé dans le traitement ou la prophylaxie d'une maladie neurodégénérative selon la revendication 8, **caractérisé en ce que** la maladie neurodégénérative est constituée par la maladie d'Alzheimer, la maladie de Parkinson, la sclérose latérale amyotrophique, la schizophrénie, la maladie de Huntington, les lésions cérébrales comme les attaques et les ischémies, la sclérose en plaques, l'épilepsie, la maladie de Friedreich, les encéphalopathies spongiformes, l'amyloïdose, la démence vasculaire, les tauophties, la maladie de Steele-Richardson, la dégénérescence cortico-basale, la dégénérescence fronto-temporale lobulaire, le parkinsonisme panencéphalitique sclérosant sous-aigu, le parkinsonisme postencéphalitique, l'encéphalite pugilistique, le complexe de parkinsonisme-démence de guam, la maladie de Pick, la démence frontotemporale, la démence associée au SIDA, la sclérose en plaques, les désordres de l'humeur tels que la dépression, la schizophrénie et les désordres bipolaires, la promotion de la récupération fonctionnelle après une attaque et une lésion cérébrale, en particulier les lésions cérébrales traumatiques.

11. Composé destiné à être utilisé dans le traitement ou la prophylaxie d'une maladie hématologique selon la revendication 8, **caractérisé en ce que** la maladie hématologique est constituée par la thalassémie, l'anémie, l'anémie aplasique, l'anémie Diamond-Blackfan, la drépanocytose, les désordres hématologique qui requièrent des transfusions régulières de globules rouges, le syndrome myélodysplasique, les disfonctionnements cardiaques induits par le fer, les insuffisances cardiaques induites par le fer et le diabète.

12. Composé destiné à être utilisé dans le traitement ou la prévention du cancer selon la revendication 9, **caractérisé en ce que** le cancer est constitué par le cancer de l'intestin, du foie, de l'estomac, du sein, du poumon, des ovaires, de la prostate, le gliome cérébral, lymphatique, de la peau, des pigments, de la glande thyroïde, la leucémie et de la moelle osseuse.

13. Composition pharmaceutique comprenant au moins un composé de formule (I) selon l'une quelconque des revendications 1 à 6, ses sels ou solvatés ou tautomères et au moins un porteur pharmaceutiquement acceptable.

14. Utilisation d'un composé de formule (I) selon l'une quelconque des revendications 1 à 6, ou tout sel ou solvaté de celui-ci, comme réactif pour des essais biologiques, de préférence comme réactif pour des essais pharmacocinétiques, des essais de traversée de la barrière cerveau-sang, des essais de chélation, des essais sur la protection contre la mort cellulaire induite par le peroxide d'hydrogène, la protection contre la mort cellulaire induite par le 6-OHDA, la neuroprotection contre la toxicité A5 et l'inhibition de la sécrétion de béta-amyloïde.
